# EUROPEAN PATENT APPLICATION

(11) **EP 1 533 613 A1**
(43) Date of publication of application: **25.05.2005**
(21) Application number: 04025210.8
(22) Date of filing: 22.10.2004
(51) Int. Cl.: G01N 33/48

(54) **Soluble transferrin receptor**

(30) Priority: 22.10.2003 EP 03023980; 06.05.2004 EP 04010822
(71) Applicant: Roche Diagnostics GmbH, 68305 Mannheim (DE); F.HOFFMANN-LA ROCHE AG, 4070 Basel (CH)
(72) Inventor: Lehmann, Paul, 67549 Worms (DE); Roeddiger, Ralf, 69517 Gorxheimertal (DE)
(74) Representative: Dey, Michael, Dipl.-Chem. Dr.

(57) **Abstract**

The invention concerns a method for detecting coronary syndromes, in particular, coronary artery disease (CAD), using risk markers.

## Description

The invention concerns a method for detecting coronary syndromes, in particular, coronary artery disease (CAD), using risk markers.

A number of markers, for example, troponin T, C-reactive protein (CRP) as well as brain natriuretic peptide (BNP), are known for diagnosing coronary diseases such as NSTEMI and acute coronary syndrome. Elevation of the concentration of one of these markers is associated with an increase in the likelihood of ischemic events including death. Further, it has already been found that CRP and troponin I or troponin T are two independent markers for risk stratification of patients suffering from acute coronary syndrome.

Since many persons are affected by coronary diseases or/and diabetes mellitus, it is desirable, however, to provide further and, above all, reliable markers for these diseases.

Therefore, it was an object of the invention to provide additional markers for coronary diseases or/and diabetes mellitus and, in particular, markers allowing an assessment of risk already at an early stage.

According to the invention this object is achieved by using sTfR (soluble transferrin receptor) or/and frataxin or/and sTfR/log ferritin (ferritin index) as a risk marker for coronary syndromes or/and diabetes mellitus. The invention, thus, relates to the use of sTfR for a novel purpose as well as to the use of the novel marker frataxin for diagnosing or prognosing coronary diseases or/and the risk of diabetes mellitus.

sTfR or/and frataxin are preferably used as markers and more preferably sTfR and frataxin and ferritin index.

It is preferred to determine additional markers for diagnosis such as a BNP peptide, a CRP peptide, a troponin peptide, hepcidin or fragments thereof, in particular, hs-CRP, hepcidin, NT-proBNP or/and troponin T.

According to the invention the above-mentioned compounds can be used as cardiac biomarkers and are strong predictors of risk among patients for acute coronary syndromes (ACS) or/and diabetes mellitus. In particular, inreased levels of the aforementioned compounds are associated with higher rates of death and recurrent ischemic events.

According to the invention it is preferred to use combinations of the aforementioned biomarkers, whereby at least one marker selected from the groups: 1) soluble transferrin receptor (sTfR) and/or frataxin and/or ferritin index, 2) CRP and/or hepcidin, 3) a BNP peptide as well as 4) a troponin peptide or fragments of those markers are used.

According to the invention it is especially preferred to use combinations of the aformentioned biomarkers, whereby at least one marker selected from the groups:
1) soluble transferrin receptor (sTfR) and/or frataxin and/or ferritin index,
2) highly sensitive C-reactive protein (hs-CRP) and/or hepcidin,
3) NT-proBNP as well as
4) troponin T
is used each. Since these biomarkers each assess different pathophysiological mechanisms in myocardial ischema, their combined use enables highly reliable diagnosis.

Elevation in sTfR is a marker for functional iron deficiency in chronic diseases. Functional iron deficiency thereby is characterized by sTfR values greater 4 mg/L. It has now been found that it is also an early prognostic marker of coronary syndromes, in particular, coronary syndromes in chronic infection and inflammation processes. sTfR is the earliest marker of anemia of chronic disease (ACD) and has now been recognized as an early risk marker for coronary artery disease (CAD). sTfR is a transmembrane protein. sTfR binds diferric transferrin, thereby delivering iron to the cytosol. In the case of an increased cellular demand for iron the sTfR expression is increased to facilitate iron uptake.

Due to its role in the metabolism sTfR can be used as a risk marker of mitochondrial dysfunction. Cytosolic iron content is regulated by the enzyme aconitase, an iron-sulfur protein. In the case of cytosolic iron decrease aconitase binds to iron-responsive element-binding protein (IRE-BP), leading to an iron uptake. The iron uptake again is downregulated by the protein frataxin. In most cases mitochondrial iron accumulation is triggered by the lack or decrease of frataxin.

Mitochondrial iron overload, in turn, causes a damage to mitochondrial functions through the iron-sulfur (Fe-S) cluster-containing subunits of the respiratory complex. An sTfR, therefore, can be used as a marker of mitochondrial dysfunction. A damage to mitochondrial functions has the following consequences:
- destabilization of iron-sulfur clusters of the mitochondrial respiratory chain
- a deficit of mitochondrial ATP production
- secretion of frataxin by the mitochondrion
- loss of aconitase activity in the cytosol reflecting a decrease of cytosolic iron content, leading to an increase in TfR and sTfR on the cell surface.

In addition to its role as a marker of mitochondrial dysfunction, sTfR has now been found to be useful also as a marker in coronary diseases, in particular, in coronary diseases on cardiac muscle cells. Mitochondrial defects, as discussed above, would preferentially be seen on tissues that generate energy by respiratory oxidation.Cardiac myocytes derive most of their ATP from the oxidation of free fatty acids. Therefore, decreased ATP generation leads to increased H₂O₂ production in cardiac muscle (so-called iron-induced oxidative stress). Therefore, mainly cardiac myocytes are victims of deficits in mitochondrial ATP production. As a consequence, cellular defects as in myocardial infarction and chronic inflammation will have an impact on the elevated concentration of sTfR. Therefore, sTfR can be used as a biochemical marker for risk stratification among patients suffering from cellular defects as occurring in mycoardial infarction and chronic inflammation. The concentration of sTfR correlates with the degree of cellular damage in the patient's tissue.

According to the invention sTfR as an independent risk marker in coronary syndromes or/and diabetes mellitus allows to determine particular diseases in patients and, thus, to determine effective therapies, e.g. effective Epo therapy. rH-Epo protects the myocardion from ischemia reperfusion injury and promotes beneficial remodelling. The therapeutic role of recombinant human Epo (rH-Epo) in the treatment of myocardial ischemia and infarction can be explained by its role in the regulation of the functional iron deficiency, its role as a tissue-protective cytokine and its role in the regulation of deficits in mitochondrial ATP production.

Preferably, amounts of > 2.5 mg/l, more preferred of > 3 mg/l and, in particular, > 4 mg/l of sTfR are considered as an indication of coronary syndromes and/or a risk of coronary syndromes and/or of diabetes mellitus and/or the risk of diabetes mellitus.

In sum, the determination of sTfR, optionally in combination with determination of ferritin for obtaining the ferritin index, is a sensitive tool for the assessment of functional iron deficiency in different patient groups. sTfR values were significantly higher in patients compared to healthy controls. Further, the assessment of sTfR allows to stratify coronary risks, in particular, among patients with chronic diseases more effectively than by established biochemical markers (heart diseases, diabetes, renal failure, rheumatoid arthritis). The assessment of sTfR, optionally in combination with the ferritin index, further allows to stratify the risk of diabetes mellitus.

In a further preferred embodiment of the invention, frataxin is used as a risk marker for coronary syndromes and/or diabetes mellitus. It was found that frataxin is one of the earliest markers of functional iron deficiency and a risk marker for coronary artery disease (CAD).

Preferably, frataxin is determined using PCR, whereby a number of trinucleotide repeats for the trinucleotide GAA after PCR of less than 10 (normal range = 10-21), in particular, of less than 9 and more preferred of less than 8 is considered as an indication of coronary syndromes and/or a risk of coronary syndromes and/or of diabetes mellitus and/or a risk of diabetes mellitus.

sTfR/log ferritin, also designated ferritin index, is the ratio of soluble transferrin receptor concentration to ferritin concentration. Values of sTfR > 4 mg/l and of ferritin > 100 µg/l reflect a functional iron deficit. Characteristic for latent iron deficit are values of ferritin < 100 µg/l while sTfR usually exceeds > 4 mg/l, without an increased risk of coronary syndromes. Thus, knowledge of the ferritin value is decisive for ferritin index evaluation. Preferably, values of the ferritin index of < 2 for CRP > 5 mg or < 3.2 for CRP < 5 mg are considered as an indication of coronary syndromes and a risk of coronary syndromes or/and of diabetes mellitus or/and a risk of diabetes mellitus.

| | Latent iron deficit | Risk of coronary syndrome (functional iron deficit) |
|---|---|---|
| sTfR | > 4 mg/l | > 4 mg/l |
| ferritin | < 100 µg/l | > 100 µg/l |
| sTfR [mg/l]/log ferritin > [µg/l] with acute phase (CRP > 5 mg/l) | 2 | < 2 |
| sTfR [mg/l]/log ferritin > [µg/l] without acute phase (CRP < 5 mg/l) | 3.2 | < 3.2 |

The invention also relates to the use of sTfR or/and frataxin or/and ferritin index as a risk marker in the manufacture of an agent to detect and/or predict coronary syndromes or/and diabetes mellitus as well as to the in vitro use of sTfR or/and frataxin or/and ferritin index as a risk marker for coronary syndromes or/and diabetes mellitus. One or both or all three of the mentioned markers are preferably combined with established cardiac biomarkers.

CRP, in particular, hs-CRP and/or hepcidin have been used primarily as a marker of systemic chronic inflammation. It is now appreciated, however, that inflammation also plays a central role in arteriosclerosis and its complications. Thus, CRP and/or hepcidin may not only reflect the degree of underlying inflammation predisposing to arteriosclerosis but also play a direct role in promoting plaque rupture and thrombosis. Preferably, amounts of > 2 mg/l (referring to blood), more preferred > 3 mg/l and most preferred > 3.5 mg/l of CRP, in particular, of hs-CRP are considered as an indication of coronary syndromes and/or a risk of coronary syndromes.

BNP peptides include BNP-32, a 32-amino acid neurohormone, preproBNP (108 amino acids), NT-proBNP (76 amino acids) as well as fragments thereof. NT-proBNP is preferred.

NT-proBNP, being part of the neurohormonal axis, is elevated in the setting of left ventricular overload. Changes in NT-proBNP concentration can be used to evaluate the success of treatment in patients with left ventricular dysfunction. NT-proBNP levels have been shown to be elevated in acute coronary syndrome, even in the absence of infarction. As ischemia may lead to a transient decrease both of systolic function and of compliance, evaluation in NT-proBNP may reflect not only the underlying impairment in left ventricular function but also the severity of the acute ischemic insult.

Preferably, amounts of > 100 pg/ml (referring to blood), more preferred > 125 pg/ml and most preferred > 150 pg/ml of a BNP peptide, in particular, of BNP, preproBNP or NT-proBNP, most preferred of NTproBNP are considered as an indication of coronary syndromes and/or a risk of coronary syndromes.

Troponin peptides include troponin I and troponin T as well as fragments thereof.

Troponin T (TnT) is a sensitive and specific marker of myocardial necrosis. A level of > 0.05 µg/l (referring to blood), in particular, of > 0.1 µg/l, preferably of > 0.2 µg/l is considered as an indication of coronary syndromes and/or a risk of coronary syndromes.

In addition to the above-mentioned markers further markers may be measured such as ischemia-modified albumin (IMA) which is a marker for mycoardial ischemia. IMA can be used in early evaluation of acute coronary syndromes (ACS) prior to heart attack in patients having chest pain suggestive of cardiac origin. Myoglobin and CK/CK-MB are markers for the degree of necrosis in heart muscle damage after myocardial infarction.

The preferred combination of markers according to the invention allows novel cardiac risk stratification in coronary syndromes and, in particular, arrangement of patient groups in different disease categories in a simple manner. Further, therapy can be proposed in a simple manner due to the information obtained by the markers. The markers of the invention and, in particular, the preferred combinations of markers also allow for a novel risk stratification of diabetes mellitus.

The markers sTfR or/and frataxin or/and ferritin index used according to the invention are also preferably used in combination with one or more markers of the following groups (i) to (iv). They are preferably used together with a marker of group (iv), which are non-specific markers of myocardial injury.

Markers of this type are characteristic, for example, for diseases associated with inflammation such as stable angina or hypertension. Examples of said markers associated with inflammation and acute phase respond include C-reactive protein, interleukin-1β, interleukin-1 receptor antagonist, interleukin-6, monocyte chemotactic protein-1, soluble intercellular adhesion molecule-1, soluble vascular cell adhesion molecule-1, tumor necrosis factor α (TNFα), caspase-3 and hemoglobin α2, whereby TNFα, IL-1 or/and IL-6 are preferably used markers according to the invention. Activation of the inflammatory response may be manifested in early stages of ACS. Therefore, measurement of the circulating concentrations of non-specific markers for inflammation and acute phase reactants can be used to identify individuals with ACS as well as individuals at risk for developing ACS.

C-reactive protein is a (CRP) is a homopentameric Ca²⁺-binding acute phase protein with 21 kDa subunits that is involved in host defense. CRP preferentially binds to phosphorylcholine, a common constituent of microbial membranes.

Phosphorylcholine is also found in mammalian cell membranes, but it is not present in a form that is reactive with CRP. The interaction of CRP with phosphorylcholine promotes agglutination and opsonization of bacteria, as well as activation of the complement cascade, all of which are involved in bacterial clearance. Furthermore, CRP can interact with DNA and histones, and it has been suggested that CRP is a scavenger of nuclear material released from damaged cells into the circulation (Robey, F.A. et al., J. Biol. Chem. 259:7311-7316, 1984). CRP synthesis is induced by II-6, and indirectly by IL-1, since IL-1 can trigger the synthesis of IL-6 by Kupffer cells in the hepatic sinusoids. The normal plasma concentration of CRP is < 3 µg/ml (30 nM) in 90% of the healthy population, and <10 µg/ml (100 nM) in 99% of healthy individuals. Plasma CRP concentrations can be measured by rate nephelometry or ELISA. The plasma concentration of CRP is significantly elevated in patients with AMI and unstable angina, but not stable angina (Biasucci, L.M. et al., Circulation 94:874-877, 1996; Biasucci, L.M. et al., Am. J. Cardiol. 77:85-87; Benamer, H. et al., Am. J. Cardiol. 82:845-850, 1998; Caligiuri, G. et al., J. Am. Coll. Cardiol. 32:1295-1304, 1998; Curzen, N.P. et al., Heart 80:23-27, 1998; Dangas, G. et al., Am. J. Cardiol. 83:583-5, A7, 1999). CRP may also be elevated in the plasma of individuals with variant or resolving unstable angina, but mixed results have been reported (Benamer, H. et al., Am. J. Cardiol. 82:845-850, 1980; Caligiuri, G. et al., J. Am. Coll. Cardiol. 32:1295-1304, 1998). CRP may not be useful in predicting the outcome of patients with AMI or unstable angina (Curzen, N.P. et al., Heart 80:23-27, 1998; Rebuzzi, A.G. et al., Am. J. Cardiol. 82:715-719, 1998; Oltrona, L. et al., Am. J. Cardiol. 80:1002-1006, 1997). The concentration of CRP will be elevated in the plasma from individuals with any condition that may elicit an acute phase response, such as infection, surgery, trauma and stroke. CRP is a secreted protein that is released into the bloodstream soon after synthesis. CRP synthesis is upregulated by IL-6, and the plasma CRP concentration is significantly elevated within 6 hours of stimulation (Biasucci, L.M. et al., Am. J. Cardiol. 77:85-87, 1996). The plasma CRP concentration peaks approximately 50 hours after stimulation, and begins to decrease with a half-life of approximately 19 hours in the bloodstream (Biasucci, L.M. et al., Am. J. Cardiol. 77:85-87, 1996). Other investigations have confirmed that the plasma CRPconcentration in individuals with unstable angina (Biasucci, L.M. et al., Circulation 94:874-877, 1996). The plasma concentration of CRP can approach 100 µg/ml (1 µM) in individuals with ACS (Biasucci, L.M. et al., Circulation 94:874-877, 1996; Liuzzo, G. et al., Circulation 94:2373-2380, 1996). CRP is a specific marker of the acute phase response. Elevations of CRP have been identified in the plasma of individuals with AMI and unstable angina, most likely as a result of activation of the acute phase response associated with atherosclerotic plaque rupture or cardiac tissue injury. CRP is a highly nonspecific marker for ACS, and elevations of the CRP concentration in plasma may occur from unrelated conditions involving activation of the immune system. Despite its high degree of non-specificity for ACS, CRP may be useful in the identification of unstable angina and AMI when used with another marker that is specific for cardiac tissue injury.

Plasma has a high concentration of CRP and there is much variability in the reported concentration of CRP in the blood of healthy individuals. Further investigation using a uniform assay, most likely a competitive immunoassay, on a range of plasma samples is necessary to determine the upper limits of the concentration of CRP in the plasma of apparently healthy individuals.

Interleukin-1β (IL-1β) is a 17 kDa secreted proinflammatory cytokine that is involved in the acute phase response and is a pathogenic mediator of many diseases. IL-1β is normally produced by macrophages and epithelial cells. IL-1β is also released from cells undergoing apoptosis. The normal serum concentration of IL-1β is < 30 pg/ml (1.8,pM). There have been no conclusive investigations into potential elevations of the plasma concentration. of IL-1β in individuals with ACS, possibly due to sensitivity limitations of the assay or clearance of IL-1β from the bloodstream soon after ACS onset. In theory, IL-1β would be elevated earlier than other acute phase proteins such as CRP in unstable angina and AMI, since IL-1β is an early participant in the acute phase response. Furthermore, IL-1β is released from cells undergoing apoptosis, which may be activated in the early stages of ischemia. In this regard, elevation of the plasma IL-1β concentration associated with ACS requires further investigation using a high-sensitivity assay. Elevations of the plasma IL-1β concentration are associated with activation of the acute phase response in proinflammatory conditions such as trauma and infection. IL-1β has a biphasic physiological half-life of 5 minutes followed by 4 hours (Kudo, S. et al., Cancer Res. 50:5751-5755,1990). IL-1β is released into the extracellular milieu upon activation of the inflammatory response or apoptosis. It is possible that IL-1β is elevated for only a short time after AMI and unstable angina episodes, and most blood samples taken on admission from patients with ACS are outside the window of IL-1β elevation following insult.

Interleukin-1 receptor antagonist (IL-1ra) is a 17 kDa member of the IL-1 family predominantly expressed in hepatocytes, epithelial cells, monocytes, macrophages, and neutrophils. IL-1ra has both intracellular and extracellular forms produced through alternative splicing. IL-1ra is thought to participate in the regulation of physiological IL-1 activity. IL-1ra has no IL-1-like physiological activity, but is able to bind the IL-1 receptor on T-cells and fibroblasts with an affinity similar to that of IL- 1β, blocking the binding of IL-1α and IL-1β and inhibiting their bioactivity (Stockman, B.J. et al., Biochemistry 31:5237-5245, 1992; Eisenberg, S.P. et al., Proc. Natl. Acad. Sci. U.S. A. 88:5232-5236, 1991; Carter, D.B. et al., Nature 344:633-638, 1990). IL-1ra is normally present in higher concentrations than IL-1 in plasma, and it has been suggested that IL-1ra levels are a better correlate of disease severity than EL- 1 (Biasucci, L.M. et al., Circulation 99:2079-2084, 1999). Furthermore, there is evidence that IL-1ra is an acute phase protein (Gabay, C. et al., J Clin. Invest. 99:29302940, 1997). The normal plasma concentration of IL-1ra is < 200 pg/ml (12 pM). The plasma concentration of IL-1ra is elevated in patients with AMI and unstable angina that proceeded to AMI, death, or refractory angina (Biasucci, L.M. et al., Circulation 99:2079-2084, 1999; Latini, R. et al., J Cardiovasc. Pharmacol. 23:1-6, 1994). Furthermore, IL-1ra was significantly elevated in severe AMI as compared to uncomplicated AMI (Latini, R. et al., J Cardiovasc. Pharmacol. 23:1-6, 1994). This indicates that IL-1ra may be a useful marker of ACS severity in unstable angina and AMI. Elevations in the plasma concentration of IL-1ra are associated with any condition that involves activation of the inflammatory or acute phase response, including infection, trauma, and arthritis. IL-1ra is released into the bloodstream in pro-inflammatory conditions, and it may also be released as a participant in the acute phase response. The major sources of clearance of IL-1ra from the bloodstream appear to be kidney and liver (Kim,.D.C. et al., J Pharm. Sci. 84:575-580, 1995). IL-1ra concentrations were elevated in the plasma of individuals with unstable angina within 24 hours of onset, and these elevations may even be evident within 2 hours of onset (Biasucci, L.M. et al., Circulation 99:2079-2084, 1999). In patients with severe progression of unstable angina, the plasma concentration of IL-1ra was higher 48 hours after onset than levels at admission, while the concentration decreased in patients with uneventful progression (Biasucci, L.M. et al., Circulation 99:2079-2084, 1999). In addition, the plasma concentration of IL-1ra associated with unstable angina can approach 1.4 ng/ml (80 pM). IL-1ra may be a useful marker of ACS severity. It is not a specific marker of ACS, but changes in the plasma concentration of IL-1ra appear to be related to disease severity. Furthermore, it is likely released in conjunction with or soon after IL-1 release in pro-inflammatory conditions, and it is found at higher concentrations than IL-1. This indicates that IL-1ra may be a useful indirect marker of IL-1 activity, which elicits the production of IL-6. Thus., IL-1ra may be useful not only in grading the severity of unstable angina and AMI, but also in the identification of the early stages of the acute phase response, before IL-6 concentrations are significantly elevated.

Interleukin-6 (IL-6) is a 20 kDa secreted protein that is a hematopoietin family proinflammatory cytokine. LL-6 is an acute-phase reactant and stimulates the synthesis of a variety of proteins, including adhesion molecules. Its major finiction is to mediate the acute phase production of hepatic proteins, and its synthesis is induced by the cytokine IL-1. IL-6 is normally produced by macrophages and T lymphocytes. The normal serum concentration of IL-6 is < 3 pg/ml (0. 15 pM). The plasma concentration of IL-6 is elevated in patients with AMI and unstable angina, to a greater degree in AMI (Biasucci, L.M. et al., Circulation 94:874-877, 1996; Manten,
A. et al., Cardiovasc. Res. 40:389-395, 1998; Biasucci, L.M. et al., Circulation 99:2079-2084, 1999). IL-6 is not sigmificantly elevated in the plasma of patients with stable angina (Biasucci, L.M. et al., Circulation 94:874-877, 1996; Manten, A. et al., Cardiovasc. Res. 40:389-395, 1998). Furthermore, IL-6 concentrations increase over 48 hours from onset IN the plasma of patients with unstable angina with severe progression, but decrease in those with uneventful progression (Biasucci, L.M. et al., Circulation 99:2079-2084, 1999). This indicates that IL-6 may be a useful indicator of disease progression. Plasma elevations of IL-6 are associated with any nonspecific proinflammatory condition such as trauma, infection, or other diseases that elicit an acute phase response. IL-6 has a half-life of 4.2 hours in the bloodstream and is elevated following AMI and unstable angina (Manten, A. et al., Cardiovasc. Res. 40:389-395, 1998). The plasma concentration of IL-6 is elevated within 8-12 hours of AMI onset, and can approach 100 pg/ml. The plasma concentration of IL-6 in patients with unstable angina was elevated at peak levels 72 hours after onset, possibly due to the severity of insult (Biasucci, L.M. et al., Circulation 94:874-877, 1996). IL-6 appears to be a sensitive marker of inflammation associated with ACS. However, it is not specific for ACS, and may be elevated in various conditions that are considered risk factors for ACS. However, IL-6 may be useful in identifying the severity of AMI or unstable angina, allowing physicians to monitor these patients closely for disease progression. Furthermore, IL-6 maybe useful in distinguishing unstable angina and AMI from stable angina.

Tumor necrosis factor α (TNFα) is a 17 kDa secreted proinflammatory cytokine that is involved in the acute phase response and is a pathogenic mediator of many diseases. TNFα is normally produced by macrophages and natural killer cells. The normal serum concentration of TNFα is < 40 pg/ml (2 pM). The plasma concentration of TNFα is elevated in patients with AMI, and is marginally elevated in patients with unstable angina (Li, D. et al., Am. Heart J 137:1145-1152, 1999; Squadrito, F. et al., Inflamm. Res. 45:14-19, 1996; Latini, R. et al., J Cardiovasc. Pharmacol. 23:1-6, 1994; Carlstedt, F. et al., J. Intern. Med. 242:361-365, 1997). Elevations in the plasma concentration of TNFα are associated with any proinflammatory condition, including trauma, stroke, and infection. TNFα has a halflife of approximately 1 hour in the bloodstream, indicating that it may be removed from the circulation soon after symptom onset. In patients with AMI, TNFα was elevated 4 hours after the onset of chest pain, and gradually declined to normal levels within 48 hours of onset (Li, D. et al., Am. Heart J 137:1145-1152, 1999). The concentration of TNFα in the plasma of AMI patients exceeded 300 pg/ml (15 pM) (Squadrito, F. et aL, inflamm. Res. 45:14-19, 1996).

Soluble intercellular adhesion molecule (sICAM-1), also called CD54, is a 85-110 kDa cell surface-bound immunoglobulin-like integrin ligand that facilitates binding of leukocytes to antigen-presenting cells and endothelial cells during leukocyte recruitment and migration. sICAM- 1 is normally produced by vascular endothelium, hematopoietic stem cells and non-hematopoietic stem cells, which can be found in intestine and epidermis. sICAM-1 can be released from the cell surface during cell death or as a result of proteolytic activity. The normal plasma concentration of sICAM-1 is approximately 250 ng/ml (2.9 nM). The plasma concentration of sICAM-1 is significantly elevated in patients with AMI and unstable angina, but not stable angina (Pellegatta, F. et al., J Cardiovasc. Pharmacol. 30:455-460, 1997; Miwa, K. et al., Cardiovasc. Res. 36:37-44, 1997; Ghaisas, N.K. et al., Am. J CardioL 80:617-619, 1997; Ogawa, H. et al., Am. J Cardiol. 83:38-42,1999). Furthermore, ICAM-1 is expressed in atherosclerotic lesions and in areas predisposed to lesion formation, so it may be released into the bloodstream upon plaque rupture (Eyama, K. et al., Circ. Res. 85:199-207,1999, Tenaglia, A.N. et al., Am. J Cardiol. 79:742-747, 1997). Elevations of the plasma concentration of sICAM-1 are associated with ischemic stroke, hjead trauma, atherosolerosis, cancer, preeclampsia, multiple sclerosis, cystic fibrosis, and other nonspecific inflammatory states (Kim, I.S., J Neurol. Sci. 137:69-78, 1996; Laskowitz, D.T. et al., J Stroke Cerebrovasc. Dis. 7:234-241, 1998). The plasma concentration of sICAM-1 is elevated during the acute stage of AMI and unstable angina. The elevation of plasma sICAM-1 reaches its peak within 9-12 hours of AMI onset, and returns to normal levels within 24 hours (Pellegatta, F. et al., J Cardiovasc. Pharmacol. 30:455-460, 1997). The plasma concentration of sICAM can approach 700 ng/ml (8 nM), in patients with ATMI (Pellgatta, F. et al., J. Cardiovasc. Pharmacol. 30:455-460,1997). sICAM-1 is elevated in the plasma of individuals with AMI and unstable angina, but it is not specific for these diseases. It may, however, be useful marker in the differentiation of AMI and unstable angina from stable angina since plasma elevations are not associated with stable angina. Interestingly, ICAM-1 is present in atherosclerotic plaques, and may be released into the bloodstream upon plaque rupture. Thus, sICAM may be useful not only as a marker of inflammation, but also plaque rupture associated with ACS.

Vascular cell adhesion molecule (VCAM), also called CD 106, is a 100- 110 kDa cell surface-bound immunoglobulin-like integrin ligand that facilitates binding of B lymphocytes and developing T lymphocytes to antigen-presenting cells during lymphocyte recruitment. VCAM is normally produced by endothelial cells, which line blood and lymph vessels, the heart, and other body cavities. VCAM-1 can be released from the cell surface during cell death or as a result of proteolytic activity. The normal serum concentration of sVCAM is approximately 650 ng/ml (6.5 nM). The plasma concentration of sVCAM-1 is marginally elevated in patients with AMI, unstable angina, and stable angina (Mulvihill, N. et al., Am. J Cardiol 83:1265-7, A9, 1999; Ghaisas, N.K. et al., Am. J. Cardiol. 80:617-619, 1997). However, sVCAM-1 is expressed in atherosclerotic lesions and its plasma concentration may correlate with the extent of atherosclerosis (liyama, K. et al., Circ. Res. 85:199-207, 1999; Peter, K. et al., Arterioscler. Thromb. Vasc. Biol. 17:505-512, 1997). Elevations in the plasma concentration of sVCAM-1 are associated with ischemic stroke, cancer, diabetes, preeclampsia, vascular injury, and other nonspecific inflammatory states (Bitsch, A. et al., Stroke 29:2129-2135, 1998; Otsuki, M. et al., Diabetes 46:2096-2101, 1997; Banks, R.E. et al., Br. J Cancer 68:122-124, 1993; Steiner, M. et al., Thromb. Haemost. 72.979-984, 1994; Austgulen, R. et al., Eur. J. Obstet. Gynecol. Reprod. Biol. 71:53-58, 1997).

Monocyte chemotactic protein-1 (MCP-1) is a 10 kDa chemotactic factor that attracts monocytes and basophils, but not neutrophils or eosiniphils. MCP-1 is normally found inequilibrium between a monomeric and homodimeric form, and it is normally produced in and secreted by monocytes and vascular endothelial cells (Yoshimura, T. et al., FEBSLett. 244:487-493, 1989; Li, Y.S. et al., Mol. Cell. Biochem. 126:61-68, 1993). MCP-1 has been implicated in the pathogenesis of a variety of diseases that involve monocyte infiltration, including psoriasis, rheumatoid arthritis, and atherosclerosis. The normal concentration of MCP-1 in plasma is < 0. 1 ng/ml. The plasma concentration of MCP-1 is elevated in patients with AMI, and may be elevated in the plasma of patients with unstable angina, but no elevations are associated with stable angina (Soejima, H. et al., J. Am. Coll. Cardiol. 34:983-988, 11- 999 - Nishiyama, K. et al., Jpn. Circ. J 62:710-712, 1998; Matsumori, A. et al., J. Mol, Cell. Cardiol. 29:419-423, 1997). Interestingly, MCP-1 also may be involved in the recruitment of monocytes into the arterial wall during atheroselerosis. Elevations of the serum concentration of MCP-1 are associated with various conditions associated with inflammation, including alcoholic liver disease, interstitial lung disease, sepsis, and systemic lupus erythematosus (Fisher, N.C. et al., Gut 45:416-420, 1999; Suga, M. et al., Eur. Respir, J. 14:376-382) 1999; Bossink, AM. et al., Blood 86:3841-3847, 1995; Kaneko, H. et al. J Rheumatol. 26:568-573, 1999). MCP-1 is released into the bloodstream upon activation of monocytes and endothelial cells. The concentration of MCP- 1 in plasma form patients with AMI has been reported to approach 1 ng/ml (100 pM), and can remain elevated for one month (Soejima, H. et al., J. Am. Coll. Cardiol. 34:983-988, 1999). The kinetics of MCP-1 release into and clearance from the bloodstream in the context of ACS are currently unknown. MCP-1 is a specific marker of the presence of a pro-inflammatory condition that involves monocyte migration. MCP-1 is not specific for ACS, but it concentration is reportedly elevated in the plasma of patients with AMI. Furthermore, MCP-1 concentrations may not be elevated in the plasma of patients with unstable angina or stable angina, which suggests that MCP-1 may be useful in discriminating AMI from unstable and stable angina.

Caspase-3, also called C-PP-32, YAMA, and apopain, is an interleukin-1β converting enzyme (ICE)-like intracellular cysteine proteinase that is activated during cellular apoptosis. Caspase-3 is present as an inactive 32 kDa precursor that is proteolytically activated during apoptosis induction into a heterodimer of 20 kDa and 11 kDa subunits (Femandes-Alnemri, T. et al., J. Biol. Chem. 269:30761-30764, 1994). Its cellular substrates include poly (ADP-ribose) polymerase (PARP) and sterol regulatory element binding proteins (SREBPs) (Liu, X. et al., J Biol. Chem. 271:13371-133376, 1996). The normal plasma concentration of caspase-3 is unknown. There are no published investigations into changes in the plasma concentration of caspase-3 associated with ACS. There are increasing amounts of evidence supporting the hypothesis of apoptosis induction in cardiac myocytes associated with ischemia and hypoxia (Saraste, A, Herz 24:189-195, 1999; Ohtsuka, T. et al., Coron. Artery Dis. 10:221-225, 1999; James, T.N., Coron. Artery Dis. 9:291-307, 1998; Bialik, S. et al., J Clin. Invest. 100:1363-1372, 1997; Long, X. et al., J Clin. Invest. 99:2635-2643, 1997). Elevations in the plasma caspase-3 concentration may be associated with any physiological event that involves apoptosis. There is evidence that suggests apoptosis is induced in skeletal muscle during and following exercise and in cerebral ischemia (Carraro, U. and Franceschi, C., Aging (Milano) 9:19-34, 1997; MacManus, J.P. et al., J Cereb. Blood Flow Metab. 19:502-510, 1999). The usefulness of caspase-3 as a marker of cardiac cell death is presently unknown, since there have been no published reports finding caspase-3 in the peripheral blood of patients with AMI. Interestingly, ischemia-induced apoptosis may have characteristics that distinguish it from other forms of apoptosis, but the induction of caspase-3 is common to all apoptotic pathways. Caspase-3 may not prove to be more useful than other cytosolic markers of cardiac cell death, since all of these markers are released following a loss of plasma membrane integrity. Evidence also suggests that cells undergoing apoptosis do not lose membrane integrity, a characteristic of necrosis, but rather, they form apoptotic bodies with intact membranes that are ultimately ingested by macrophages and other adjacent cells (Saraste, A., Herz, 24: 1 S9-195, 1999; James, T.N., Coron. Artery Dis. 9:291-307, 1998). In this regard, the release of intracellular contents may be a result of necrosis, and caspase-3 may not be a suitable marker for the identification of cardiac cell death, particularly as a result of apoptosis.

Hemoglobin (Hb) is an oxygen-carrying iron-containing globular protein found in erythrocytes. It is a heterodimer of two globin subunits. a₂γ₂ is referred to as fetal Hb, α₂β₂ is called adult HbA, and α₂δ₂ is called adult HbA₂. 90-95% of hemoglobi is HbA, and the α₂ globin chain is found in all Hb types, even sickle cell hemoglobin. Hb is responsible for carrying oxygen to cells throughout the body. Hbα₂ is not normally detected in serum. The usefulness of 1Hbα₂ on a ACS panel would be to deterniffle the extent of hemolysis and the resulting contribution of erythrocyteonginated proteins to the measured serum concentration. An accepted level of hemolysis would have to be established for the measurement of serum markers that are present in erythrocytes.

Human lipocalin-type prostaglandin D synthase (hPDGS), also called β-trace, is a 30 kDa gglycoprotein that catalyzes the formation of prostaglandin D2 from prostaglandin H. The upper limit of hPDGS concentrations in apparently healthy individuals is reported to be approximately 420 ng/ml (Patent No. EP0999447A1). Elevations of hPDGS have been identified in blood from patients with unstable angina and cerebral infarction (Patent No. EP0999447A1). Furthermore, hPDGS appears to be a useful marker of ischemic episodes, and concentrations of hPDGS were found to decrease over time in a patient with angina pectoris following percutaneous transluminal coronary angioplasty (PTCA), suggesting that the hPGDS concentration decreases as ischemia is resolved (Patent No. EP0999447A1).

Markers of group (iii) are non-specific markers for myocardial injury related to atherosclerotic plaque rupture. Markers of this type are indicative, in particular, for diseases associated with plaque rupture such as artherosclerosis or unstable angina. These, too, are very early markers, whereby the appearance of markers related to artherosclerotic plaque rupture may precede specific markers of myocardial injury when ACS is due to atherosclerotic plaque rupture. Potential markers of atherosclerotic plaque rupture include human neutrophil elastase, inducible nitric oxide synthase, lysophosphatidic acid, malondialdehyde-modified low density lipoprotein and various members of the matrix metalloproteinase (MMP) family, including MMP-1, MMP-2, MMP-3 and MMP-9. According to the invention MMP is preferably used as a further marker.

Human neutrophil elastase (HNE) is a 30 kDa serine proteinase that is normally contained within the azurophilic granules of neutrophils. HNE is released upon neutrophil activation, and its activity is regulated by circulating a1-proteinase inhibitor. Activated neutrophils are commonly found in atherosclerotic plaques, and rupture of these plaques may result in the release of HNE. The plasma HNE concentration is usually measured by detecting HNE-α₁-PI complexes. The normal concentration of these complexes is 50 ng/ml, which indicates a normal concentration of approximately 2:5 ng/ml (0.8 nM) for HNE. HNE release also can be measured through the specific detection of fibrinopeptide Bb30-43, a specific HINE-derived fibrinopeptide, in plasma. Plasma HNE is elevated in patients with coronary stenosis, and its elevation is greater in patients with complex plaques than those with simple plaques (Kosar, F. et al., Angiology 49:193-201, 1998- Amaro, A. et al., Eur. Heart J. 16:615-622, 1995). Plasma HNE is not significantly elevated in patients with stable angina, but is elevated in patients with unstable angina and AMI, as determined by measuring fibrinopeptide Bb30-43 with concentrations in unstable angina being 2.5-fold higher than those associated with AMI (Dinerman, J.L. et al., J Am. Coll. Cardiol. 15:1559-1563, 1990; Mehta, J. et al., Circulation 79:549-556, 1989). Serum HNE is elevated in cardiac surgery, exercise-induced muscle damage, giant cell arteritis, acute respiratory distress syndrome, appendicitis, pancreatitis, sepsis, smoking-associated emphysema, and cystic fibrosis (Genereau, T. et al., J Rheumatol. 25:710-713, 1998; Mooser, V. et al.,Arterioscler. Thromb. Vasc. Biol. 19:1060-1065, 1999; Gleeson, M. et al., Eur. J. Appl. Physiol. 77:543-546, 1998; Gando, S. et al., J. Trauma 42:1068-1072, 1997; Eriksson, S. et al., Eur. J. Surg. 161:901-905, 1995; Liras, G. et al., Rev. Esp. Enferm. Dig. 87:641-652, 1995; Endo, S. et al., J. Inflamm. 45:136-142, 1995; Janoff, A., Annu Rev Med 36:207-216, 1985). HNE may also be released during blood coagulation (Plow, E.F. and Plescia, J., Thromb, Haemost. 59:360-363, 1988; Plow, E.F., J Clin. Invest. 69:564-572, 1982). Serum elevations of HNE could also be associated with any non-specific infection or inflammatory state that involves neutrophil recruitment and activation. It is most likely released upon plaque rupture, since activated neutrophils are present in atherosclerotlie plaques. HNE is presumably cleared by the liver after it has formed a complex with α₁-PI.

Inducible nitric oxide synthase (iNOS) is a 130 kDa cytosolic protein in epithelial cells macrophages whose expression is regulated by cytokines, including interferon-γ, interleukin-1β, interleukin-6, and tumor necrosis factor α, and lipopolysaccharide. iNOS catalyzes the synthesis of nitric oxide (NO) from L-arginine, and its induction results in a sustained high-output production of NO, which has antimicrobial activity and is a mediator of a variety of physiological and inflammatory events. NO production by iNOS is approximately 100 fold more than the amount produced by constitutively-expressed NOS (Depre, C. et al., Cardiovasc. Res. 41:465472, 1999). There are no published investigations of plasma iNOS concentration changes associated with ACS. iNOS is expressed in coronary atherosclerotic plaque, and it may interfere with plaque stability through the production of peroxynitrate, which is a product of NO and superoxide and enhances platelet adhesion and aggregation (Depre, C. et al., Cardiovasc. Res. 41:465-472, 1999). iNOS expression during cardiac ischemia may not be elevated, suggesting that iNOS maybe useful in the differentiation of angina from AMI (Hammerman, S.I. et al., Am. J Physiol. 277:H1579-H1592,1999; Kaye, RM. et al., Life Sci 62:883-887,1998). Elevations in the plasma iNOS concentration may be associated with cirrhosis, iron-deficiency anemia, or any other condition that results in macrophage activation, including bacterial infection (Jimenez, W. et al., Hepatology 30:670-676, 1999; Ni, Z. et al., Kidney Int. 52:195-201, 1997). NOS may be released into the bloodstream as a result of atheroselerotic plaque rupture, and the presence of increased amounts of iNOS in the bloodstream may not only indicate that plaque rupture has occurred, but also that an ideal environment has been created to promote platelet adhesion. However, iNOS is not specific for atherosclerotic plaque rupture, and its expression can be induced during non-specific inflammatory conditions.

Lysophosphatidic acid (LPA) is a lysophospholipid intermediate formed in the synthesis of phosphoglycerides and triacylglycerols. It is formed by the acylation of glycerol-3 phosphate by acyl-coenzyme A and during mild oxidation of low-density lipoprotein (LDL). LPA is a lipid second messanger with vasoactive properties, and it can function as a platelet activator. LPA is a component of atherosclerotic lesions, particularly in the core, which is most prone to rupture (Siess, W., Proc. Natl. Acad. Sci. U. S. A. 96, 6931-6936, 1999). The normal plasma LPA concentration is 540 nM. Serum LPA is elevated in renal failure and in ovarian cancer and other gynecologic cancers (Sasagawa, T. et al., J. Nutr. Sci. Vitaminol. (Tokyo) 44:809-818,1998; Xu, Y. et al., JAMA 280:719-723, 1998). In the context of unstable angina, LPA is most likely released as a direct result of plaque rupture. The plasma LPA concentration can exceed 60 µM in patients with gynecologic cancers (Xu, Y. et al., JAMA 280:719-723, 1998). Serum LPA may be a useful marker of atherosclerotic plaque rupture, which may allow the discrimination of unstable angina from stable angina. However, LPA may not be as specific as other markers of plaque rupture.

Malondialdehyde-modified low-density lipoprotein (MDA-modified LDL) is formed during the oxidation of the apoB-100 moiety of LDL as a result of phospholipase activity, prostaglandin synthesis, or platelet activation. MDA-modified LDL can be distinguished from oxidized LDL because MDA modifications of LDL occur m the absence of lipid peroxidation (Holvoet, P., Acta Cardiol. 53:253-260, 1998). The normal plasma concentration of MDA-modified LDL is less than 4 µg/ml (~10 µM). Plasma concentrations of oxidized LDL are elevated in stable angina, unstable angina, and AMI, indicating that it may be a marker of atheroselerosis (Holvoet, P, Acta Cardiol. 53:253-260, 1998; Holvoet, P. et al., Circulation 98:14871494, 1998). Plasma MDA-modified LDL is not elevated in stable angina, but is significantly elevated in unstable angina and AMI (Holvoet, P., Acta Cardiol. 53:253260, 1998; Holvoet, P. et al., Circulation 98:1487-1494, 1998; Holvoet, P. et al., JAMA 281.1718-1721, 1999). Plasma MDA-modified LDL is elevated in individuals with beta-thallasemia and in renal transplant patients (Livrea, M.A. et al., Blood 92:39363942, 1998; Ghanem, H. et al., Kidney Int. 49:488-493, 1996; van den Dorpel, M.A. et al., Transpl. Int. 9 Suppl. 1:S54-S57, 1996). Furthermore, serum MDA-modified LDL may be elevated during hypoxia (Balagopalakrishna, C. et al., Adv. Exp. Med. Biol. 411:337-345, 1997). The plasma concentration of MDA-modified LDL is elevated within 6-8 hours from the onset of chest pain. Plasma concentrations of MDA-modified LDL can approach 20 µg/ml (~50 µM) in patients with AMI, and 15 µg/ml (~40 µM) in patients with unstable angina (Holvoet, P. et al., Circulation 98: 1487-1494, 1998). Plasma MDA-modified LDL has a half-life of less than 5 minutes in mice (Ling, W. et al., J Clin. Invest. 100:244-252, 1997). MDA-modified LDL appears to be a specific marker of atherosclerotic plaque rupture in acute coronary symptoms. II is unclear. however, if elevations in the plasma concentration of MDA-modified LDL are a result of plaque rupture or platelet activation. The most reasonable explanation is that the presence of increased amounts of MDA-modified. LDL is an indication of both events. MDA-modified LDL may be useful in discriminating unstable angina and AMI from stable angina, but it alone can not distinguish AMI from unstable angina. In this regard, MDA-modified LDL would be most useful as part of a panel of markers, particularly with another marker that can distinguish AMI from unstable angina.

Matrix metalloproteinase-1 also called collagenase-1, is a 41/44 kDa zinc- and calcium-binding proteinase that cleaves primarily type I collagen, but can also cleave collagen types II, III, VII and X. The active 41/44 kDa enzyme can undergo autolysis to the still active 22/27 kDa form. MMP-1 is synthesized by a variety of cells, including smooth muscle cells, mast cells, macrophage-derived foam cells, T lymphocytes, and endothelial cells (Johnson, J. L. et al., Arterioscler. Thromb. Vasc. Biol. 18:1707- 1715, 1998). MMP- 1, like other MMPs, is involved in extracellular matrix remodeling, which can occur following injury or during intervascular cell migration. MMP-1 can be found in the bloodstream either in a free form or in complex with TIMP- 1, its natural inhibitor. MMP- 1 is normally found at a concentration of < 25 ng/ml in plasma. There have been no conclusive published investigations into changes in the serum or plasma concentration of MMP-1 associated with ACS. However, MMP-1 is found in the shoulder region of atheroselerotic plaques, which is the region most prone to rupture, and may be involved in atherosclerotic plaque destabilization (Johnson, J.L. et al., Arterioscler. Thromb. Vasc. Biol. 18: 1707-1715, 1998). Furthermore, MMP-1 has been implicated in the pathogenesis of myocardial reperfusion injury (Shibata, M. et al., Angiology 50:573582. 1999). Serum MMP-1 may be elevated inflammatory conditions that induce mast cell degranulation. Serum MMP-1 concentrations are elevated in patients with arthritis and systemic lupus erythematosus (Keyszer, G. et al., Z Rheumatol 57:392-398, 1998; Keyszer, G. J Rheumatol. 26:251-258, 1999). Serum MMP-1 also is elevated in patients with prostate cancer, and the degree of elevation corresponds to the metastatic potential of the tumor (Baker, T. et al., Br. J. Cancer 70:506-512, 1994). The serum concentration of MMP-1 may also be elevated in patients with other types of cancer. Serum MMP-1 is decreased in patients with hemochromatosis and also in patients with chronic viral hepatitis, where the concentration is inversely relaled to the severity (George, D.K. et aL, Gut 42:715-720, 1998; Murawaki, Y. et al., J Gastroenterol. Hepatol. 14:138-145, 1999). MMP-1 is released during mast cell degranulation, and is presumably released during atherosclerotic plaque rupture. MMP-1 concentrations are lower in heparinized plasma than in EDTA plasma or serum, and diluted samples give higher concentration values in an ELISA assay than undiluted samples, presumably due to reduction of the inihibitory effects of protein MMP inhibitors or matrix components (Lein, M. et al., Clin. Biochem. 30:491-496, 1997). Serum MMP-1 was decreased in the first four days following AMI, and increased thereafter, reaching peak levels 2 weeks after the onset of AMI (George, DX. et al., Gut 42:715-720, 1998).

Matrix metalloproteinase-2 (MMP-2), also called gelatinase A, is a 66 kDa zinc- and calcium-binding proteinase that is synthesized as an inactive 72 kD a precursor. Mature MMP-3 cleaves type 1 gelatin and collagen of types IV, V, VII, and X. MMP-2 is synthesized by a variety of cells, including vascular smooth muscle cells, mast cells, macrophage-derived foam cells, T lymphocytes, and endothelial cells (Johnson, J.L. et al., Arterioscler. Thromb. Vasc. Biol. 18:1707~1715, 1998). MMP-2 is usually found in plasma in complex with TIMP-2, its physiological regulator (Murawaki, Y. et al., J. Hepatol. 30:1090-1098, 1999). The normal plasma concentration of MMP-2 is < ~550 ng/ml (8 nM). MMP-2 expression is elevated in vascular smooth muscle cells within atheroselerotic lesions, and it may be released into the bloodstream in cases of plaque instability (Kai, H. et al., J. Am. Coll. Gardiol. 32:368-372. 1998). Furthermore, MMP-2 has been implicated as a contributor to plaque instability and rupture (Shah, P.K. et al., Circulation 92:1565-1569, 1995). Serum MMP-2 concentrations were elevated inpatients with stable angina, unstable angina, and AMI, with elevations being significantly greater in unstable angina and AMI than in stable angina (Kai, H. et al., J. Am. Coll. Cardiol. 32:368-372, 1998). There was no change in the serum MMP-2 concentration in individuals with stable angina following a treadmill exercise test (Kai, H. et al., J. Am. Coll. Cardiol. 32:368-372,1998). Serum and plasma MMP-2 is elevated in patients with gastric cancer, hepatocellular carcinoma, liver cirrhosis, urothelial carcinoma, rheumatoid arthritis, and lung cancer (Murawaki, Y, et al., J. Hepatol. 30:1090-1098, 1999; Endo, K, et al., Anticancer Res. 17:2253-2258, 1997; Gohji, K. et al., Cancer 78:2379-2387, 1996; Gruber, B.L. et al., Clin. Immunol. Immunopathol. 78:161-171, 1996; Garbisa, S. et al., Cancer Res. 52:4548-4549, 1992). Furthermore, MMP-2 may also be translated from the platelet cytosol to the extracellular space during platelet aggregation (Sawicki, G. et al., Thromb. Haemost. 80:836-839, 1998). MMP-2 was elevated on admission in the serum of individuals with unstable angina and AMI, with maximum levels approaching 1.5 µg/ml (25 nM) (Kai, H. et al., J.

Am. Coll. Cardiol. 32:368-372,1998). The serum MMP-2 concentration peaked 1-3 days after onset in both unstable angina and AMI, and started to return to normal after 1 week (Kai, H. et al., J. Am. Coll. Cardiol. 32:368-372, 1998).

Matrix metalloproteinase-3 (MMP-3), also called stromelysin-1, is a 45 kDa zinc- and calcium-binding proteinase that is synthesized as an inactive 60 kDa precursor. Mature MMP-3 cleaves proteoglycan, fibrinectin, laminin, and type IV collagen, but not type I collagen. MMP-3 is synthesized by a variety of cells, including smooth muscle cells, mast cells, macrophage-derived foam cells, T lymphocytes, and endothelial cells (Johnson, J.L. et al., Arterioscler. Thromb. Vasc. Biol. 18: 1707-1715, 1998). MMP-3, like other MMPs, is involved in extracellular matrix remodeling, which can occur following injury or during intervascular cell migration. MMP-3 is normally found at a concentration of < 125 ng/ml in plasma. The serum MMP-3 concentration also has been shown to increase with age, and the concentration in males is approximately 2 times higher in males than in females (Manicourt, D.H. et al., Arthritis Rheum. 37:1774-1783, 1994). There have been no conclusive published investigations into changes in the serum or plasma concentration of MMP-3 associated with ACS. However, MMP-3 is found in the shoulder region of atherosclerotic plaques, which is the region most prone to rupture, and may be involved in atheroselerotic plaque destabilization (Johnson, J. L. et al., Arterioscler. Thromb. Vasc. Biol. 18: 1707-1715, 1998). Therefore, MMP-3 concentration maybe elevated as a result of atherosclerotic plaque rupture in unstable angina. Serum MMP-3 may be elevated inflammatory conditions that induce mast cell degranulation. Serum MMP-3 concentrations are elevated in patients with arthritis and systemic lupus erythematosus (Zucker, S. et al. J. Rheumatol. 26:78-80, 1999; Keyszer, G. et al., Z Rheumatol. 57:392-398, 1998; Keyszer, G. et al. J Rheumatol. 26:251-258, 1999). Serum MMP-3 also is elevated in patients with prostate and urothelial cancer, and also glomerulonephritis (Lein, M. et al., Urologe A 37:377-3 81, 1998; Gohji, K. et al., Cancer 78:2379-2387,1996; Akiyama, K. et al., Res. Commun. Mol.

Pathol. Pharmacol. 95:115-128, 1997). The serum concentration of MMP-3 may also be elevated in patients with other types of cancer. Serum MMP-3 js decreased in patients with hemochromatosis (George, D.K. et al., Gut 42:715-720, 1998).

Matrix metalloproteinase-9 (MMP-9) also called gelatinase B, is an 84 kDa zinc- and calcium-binding proteinase that is synthesized as an inactive 92 kDa precursor. Mature MMP-9 cleaves gelatin types 1 and V, and collagen types 1V and V. MMP-9 exists as a monomer, a homodimer, and a heterodimer with a 25 kDa α₂-microglobulin-related protein (Triebel, S. et al., FEBS Lett. 314:3 86-3 8 8, 1992). MMP-9 is synthesized by a variety of cell types, most notably by neutrophils. The normal plasma concentration of MMP-9 is < 35 ng/ml (400 pM). MMP-9 expression is elevated in vascular smooth muscle cells within atheroselerotic lesions, and it may be released into the bloodstream in cases of plaque instability (Kai, H. et al., J. Am. Coll. Cardiol. 32:368-372, 1998). Furthermore, MMP-9 may have a pathogenic role in the development of ACS (Brown, D.L. et al., Circulation 91:2125-2131, 1995). Plasma MMP-9 concentrations are significantly elevated in patients with unstable angina and AMI, but not stable angina (Kai, H. et al., J. Am. Coll. Cardiol. 32:3 68-372, 1998). The elevations in patients with AMI may also indicate that those individuals were suffering from unstable angina. Elevations in the plasma concentration of MMP-9 may also be greater in unstable angina than in AMI, but these differences may not be statistically significant. There was no significant change in plasma MMP-9 levels after a treadmill exercise test in patients with stable angina (Kai, H. et al., J. Am. Coll. Cardiol. 32:368-372, 1998). Plasma MMP-9 is elevated in individuals with rheumatoid arthritis, septic shock, giant cell arteritis and various carcinomas (Graber, B.L. et al., Clin. Immunol. Immunopathol. 78:161-171, 1996; Nakamura, T. et al., Am. J. Med. Sci. 316:3 55-360, 1998; Blankaert, D. et al., J. Acquir. Immune Defic. Syndr. Hum. Retrovirol. 18:203-209, 1998; Endo, K. et al.. Anticancer Res. 17:2253-2258, 1997; Hayasaka, A. et al., Hepatology 24:1058-1062,1996; Moore, D.H. et al., Gynecol. Oncol. 65:78-82, 1997; Sorbi, D. et al., Arthritis Rheum. 39:1747-1753, 1996; lizasa, T. et al., Clin., Cancer Res.. 5: 149-153, 1999). Furthermore, the plasma MMP-9 concentration may be elevated in stroke and cerebral hemorrhage (Mun-Bryce, S. and Rosenberg, G.A., J. Cereb. Blood Flow Metab, 18: 1163-1172, 1998; Romanic, A.M. et al., Stroke 29:1020-1030, 1998; Rosenberg, G.A., J. Neurotrauma 12:833-842, 1995). MMPP-9 was elevated on admission in the serum of individuals with unstable angina and AMI, with maximum levels approaching 150 ng/ml (1.7 nM) (Kai, H. et al, J. Am. Coll. Cardiol. 32:368-372, 1998). The serum MMP-9 concentration was highest on admission in patients unstable angina, and the concentration decreased gradually after treatment, approaching baseline more than 1 week after onset (Kai, H., et al., J. Am. Coll. Cardiol. 32:368-372, 1998).

Further, it is preferred according to the invention to use a marker of group (ii), namely a non-specific marker for myocardial injury related to coagulation. Examples of such markers are plasmin, β-thromboglobulin (β-TG), PF4, FPA, PDGF, prothrombin fragment 1+2, P-selection, thrombin, D-dimer, von Willebrand factor, TF and coagulation cascade.

There are essentially two mechanisms that are used to halt or prevent blood loss following vessel injury. The first mechanism involves the activation of platelets to facilitate adherence to the site of vessel injury. The activated platelets then aggregate to form a platelet plaque that reduces or temporarily stops blood loss. The process of platelet aggregation, plaque formation and tissue repair are all accelerated and enhanced by numerous factors secreted by activated platelets. Platelet aggregation and plaque formation is mediated by the formation of fibrinogen bridge between activated platelets. On current activation of the second mechanism, the coagulation cascade results in the generation of fibrin from fibrinogen and the formation of an insoluble fibrin clot that strengthens the platelet plaque. The markers of group (ii) are coagulation factors which are indicative, in particular, of conditions associated with platelet activation, e.g. atherosclerosis and unstable angina.

Plasmin is a 78 kDa serine proteinase that proteolytically digests crosslinked fibrin, resulting in clot dissolution. The 70 kDa serine proteinase inhibitor α2-antiplasmin (α2AP) regulates plasmin activity by forming a covalent 1:1 stoichiometric complex with plasmin. The resulting ~150 kDa plasmin-α2AP complex (PAP), also called plasmin inhibitory complex (PIC) is formed immediately after α2AP comes in contact with plasmin that is activated during fibrinolysis. The normal serum concentration of PAP is <1 µg/ml (6.9 nM). Elevations in the serum concentration of PAP can be attributed to the activation of fibrinolysis. Elevations in the serum concentration of PAP may be associated with clot presence, or any condition that causes or is a result of fibrinolysis activation. These conditions can include atherosclerosis, disseminated intravascular coagulation, AMI, surgery, trauma, unstable angina, stroke, and thrombotic thrombocytopenic purpura. PAP is formed immediately following proteolytic activation of plasmin. PAP is a specific marker for fibrinolysis activation and the presence of a recent or continual hypercoagulable state. It is not specific for ACS and can be elevated in many other disease states.

β-thromboglobulin (βTG) is a 36 kDa platelet α granule component that is released upon platelet activation. The normal plasma concentration of βTG is < 40 ng,/ml (1.1 nM). Plasma levels of β-TG appear to be elevated in patients with unstable angina and AMI, but not stable angina (De Caterina, R. et al., Eur. Heart J. 9:913-922, 1988; Bazzan, M. et al., Cardiologia 34, 217-220, 1989). Plasma β-TG elevations also seem to be correlated with episodes of ischemia in patients with unstable angina (Sobel, M. et al., Circulation 63:300-306, 1981). Elevations in the plasma concentration of βTG may be associated with clot presence, or any condition that causes platelet activation. These conditions can include atheroselerosis, disseminated intravascular coagulation, surgery, trauma, and thrombotic thrombocytopenic purpura, and stroke (Landi, G. et al., Neurology 37:1667-1671, 1987). βTG is released into the circulation immediately after platelet activation and aggregation. It has a biphasic half-life of 10 minutes, followed by an extended 1 hour half-life in plasma (Switalska, H.I. et al., J. Lab. Clin. Med. 106:690-700, 1985). Plasma βTG concentration is reportedly elevated dring unstable angina and AMI, but these studies may not be completely reliable. Special precautions must be taken to avoid platelet activation during the blood sampling process. Platelet activation is common during regular blood sampling, and could lead to artificial elevations of plasma βTG concentration. In addition, the amount of βTG released into the bloodstream is dependent on the platelet count of the individual, which can be quite variable. Plasma concentrations of βTG associated with ACS can approach 70 ng/ml (2 nM), but this value may be influenced by platelet activation during the sampling procedure.

Platelet factor 4 (PF4) is a 40 kDa platelet α granule component that is released upon platelet activation. PF4 is a marker of platelet activation and has the ability to bind and neutralize heparin. The normal plasma concentration of PF4 is < 7 ng/ml (175 pM). The plasma concentration of PF4 appears to be elevated in patients with AM1 and unstable angina, but not stable angina (Gallino, A. et al., Am. Heart J. 112:285-290, 1986; Sakata, K. et al., Jpn. Circ. J. 60:277-284, 1996; Bazzan, M. et al., Cardiologia 34:217-220, 1989). Plasma PF4 elevations also seem to be correlated with episodes of ischemia in patients with unstable angina (Sobel, M. et al., Circulation 63:300-306, 1981). Elevations in the plasma concentration of PF4 may be associated with clot presence, or any condition that causes platelet activation. These conditions can include atherosclerosis, disseminated intravascular coagulation, surgery, trauma, thrombotic thrombocytopenic purpura, and acute stroke (Carter, AM et al., Arterioscler. Thromb. Vasc. Biol. 18: 1124-1131, 1998). PF4 is released into the circulation immediately after platelet activation and aggregation. It has a biphasic half-life of 1 minute, followed by an extended 20 minute half-life in plasma. The half-life of PF4 in plasma can be extended to 20-40 minutes by the presence of heparin (Rucinski, B. et al., Am. J. Physiol. 251:H800-H807, 1986). Plasma PF4 concentration is reportedly elevated during unstable angina and AMI, but these studies may not be completely reliable. Special precautions must be taken to avoid platelet activation during the blood sampling process. Platelet activation is common during regular blood sampling, and could lead to artificial elevations of plasma PF4 concentration. In addition, the amount of PF4 released into the bloodstream is dependent on the platelet count of the individual, which can be quite variable. Plasma concentrations of PF4 associated with disease can exceed 100 ng/ml (2.5 nM), but it is likely that this value may be influenced by platelet activation during the sampling procedure.

Fibrinopeptide A (FPA) is a 16 amino acid, 1. 5 kDa peptide that is liberated from amino terminus of fibrinogen by the action of thrombin. Fibrinogen is synthesized and secreted by the liver. The normal plasma concentration of FPA is < 5 ng/ml (3.3 nM). The plasma FPA concentration is elevated in patients vrith AMI, unstable angina, and variant angina, but not stable angina (Gensini, G.F. et al., Thromb.Res. 50:517-525, 1988; Gallino, A. et al., Am. Heart J. 112:285-290, 1986: Sakata, K. et al., Jpn. Circ. J. 60:277-284, 1996; Theroux, P. et al., Circulation 75:156-162, 1987; Merlini, P.A. et al., Circulation 90:61-68, 1994; Manten, A. et al., Cardiovasc. Res. 40:3 ) 89-395, 1998). Furthermore, plasma FPA may indicate the severity of angina (Gensini, G.F. et al., Thromb. Res. 50:517-525, 1988). Elevations in the plasma concentration of FPA are associated with any condition that involves activation of the coagulation pathway, including stroke, surgery, cancer, disseminated intravascular coagulation, nephrosis, and thrombotic thrombocytopenic purpura. FPA is released into the circulation following thrombin activation and cleavage of fibrinogen. Because FPA is a small polypeptide, it is likely cleared from the bloodstream rapidly. FPA has been demonstrated to be elevated for more than one month following clot formation, and maximum plasma FPA concentrations can exceed 40 ng/ml in active angina (Gensini, G.F. et al., Thromb. Res. 50:517-525, 1988; Tohgi, H. et al., Stroke 21:16631667, 1990).

Platelet-derived growth factor (PDGF) is a 28 kDa secreted homo- or heterodimeric protein composed of the homologous subunits A and/or B (Mahadevan, D. et al., J. Biol. Chem. 270:27595-27600, 1995). PDGF is a potent mitogen for mesenchymal cells, and has been implicated In the pathogenesis of atherosclerosis. PDGF is released by aggregating platelets and monocytes near sites of vascular injury. The normal plasma concentration of PDGF is < 0.4 ng/ml (15. pM). Plasma PDGF concentrations are higher in individuals with AMI and unstable angina than in healthy controls or individuals with stable angina (Ogawa, H. et al., Am. J. Cardiol. 69:453456, 1992; Wallace, J.M, et al., Ann. Clin. Biochem. 35:236-241, 1998; Ogawa, H. et al., Coron. Artery Dis. 4:437-442, 1993). Changes in the plasma PDGF concentration in these individuals is most likely due to increased platelet and monocyte activation. Plasma PDGF is elevated in individuals with brain tumors, breast cancer, and hypertension (Kurimoto, M. et al., Acta Neurochir. (Wien) 137:182-187, 1995; Seymour, L. et al., Breast Cancer Res. Treat. 26:247-252, 1993; Rossi, E. et al., Am. J. Hypertens. 11: 1239-1243, 1998). Plasma PDGF may also be elevated in any proinflammatory condition or any condition that causes platelet activation including surgery, trauma, disseminated intravascular coagulation, and thrombotic thrombocytopenic purpura. PDGF is released from the secretory granules of platelets and monocytes upon activation. PDGF has a biphasic half-life of approximately 5 minutes and 1 hour in animals (Cohen, A.M et al., J. Surg. Res. 49:447-452, 1990; Bowen-Pope, D.F. et al., Blood 64:458-469, 1984). The plasma PDGF concentration in ACS can exceed 0.6 ng/ml (22 pM) (Ogawa, H. et al., Am. J. Cardiol. 69:453-456, 1992). PDGF may be a sensitive and specific marker of platelet activation. In addition, it may be a sensitive marker of vascular injury, and the accompanying monocyte and platelet activation.

Prothrombin fragment 1+2 is a 32 kDa polypeptide that is liberated from the amino teminus of thrombin during thrombin activation. The normal plasma concentration of F1+2 is < 32 ng/ml (1 nM). Reports from investigations of plasma F1+2 concentration elevations that are associated with ACS are conflicting. The plasma concentration of F1+2 is reportedly elevated in patients with AMI and unstable angina, but not stable angina, but the changes were not robust (Merlini, P.A. et al., Circulation 90:61-68, 1994). Other reports have indicated that there is no significant change in the plasma F1+2 concentration in cardiovascular disease (Biasucci, L.M. et al., Circulation 93:2121-2127, 1996; Manten, A. et al., Cardiovasc. Res. 40:389-395, 1998). The concentration of F1+2 in plasma can be elevated during any condition associated with coagulation activation, including stroke, surgery, trauma, thrombotic thrombocytopenic purpura, and disseminated intravascular coagulation. F1+2 is released into the bloodstream immediately upon thrombin activation. F1+2 has a halflife of approximately 90 minutes in plasma, and it has been suggested that this long half-life may mask bursts of thrombin formation (Biasucci, LM. et al., Circulation 93:2121-2127, 1996).

P-selectin, also called granule membrane protein-140, GMP-140, PADGEM, and CD-62P, is a ~140 kDa adhesion molecule expressed in platelets and endothelial cells. P-selectin is stored in the alpha granules of platelets and in the Weibel-Palade bodies of endothelial cells. Upon activation, P-selectin is rapidly translocated to the surface of endothelial cells and platelets to facilitate the "rolling" cell surface interaction with neutrophils and monocytes. Membrane-bound and soluble forms of P-selectin have been identified. Soluble P-selectin may be produced by shedding of membrane-bound P-selectin either by proteolysis of the extracellular Pselectin molecule, or by proteolysis of components of the intracellular cytoskeleton in close proximity to the surface-bound P-selectin molecule (Fox, J.E., Blood Coagul. Fibrinolysis 5:291-304, 1994). Additionally, soluble P-selectin may be translated from mRNA that does not encode the N-terminal transmembrane domain (Dunlop, L.C. et. al., J. Exp. Med. 175:1147-1150, 1992; Johnston, G.I. et al., J. Biol. Chem. 265:2138121385, 1990). Activated platelets can shed membrane-bound P-selectin and remain in the circulation, and the shedding of P-selectin can elevate the plasma P-selectin concentration by approximately 70 ng/ml (Michelson, A.D. et al., Proc. Natl. Acad. Sci. U. S. A. 93:11877-11882, 1996). Soluble P-selectin may also adopt a different conformation than membrane-bound P-selectin. Soluble P-selectin has a monomeric rod-like structure with a globular domain at one end, and the membrane-bound molecule forms rosette structures with the globular domain facing outward (Ushiyama, S. et al., J. Biol. Chem. 268:15229-15237, 1993). Soluble P-selectin may play an important role in regulating inflammation and thrombosis by blocking interactions between leukocytes and activated platelets and endothelial cells (Gamble, J.R. et al., Science 249:414-417, 1990). The normal plasma concentration of soluble P-selectin is < 200 ng/ml. Blood is normally collected using citrate as an anticoagulant, but some studies have used EDTA plasma with additives such as prostaglandin E to prevent platelet activation. EDTA may be a suitable anticoagulant that will yield results comparable to those obtained using citrate. Furthermore, the plasma concentration of soluble P-selectin may not be affected by potential platelet activation during the sampling procedure. The plasma soluble P-selectin concentration was significantly elevated in patients with AMI and unstable angina, but not stable angina, even following an exercise stress test (Ikeda, H. et al., Circulation 92:1693-1696, 1995; Tomoda, H. and Aoki, N., Angiology 49:8 07-813, 1998; Hollander, J.E. et al., J. Am. Coll. Cardiol. 34:95-105, 1999; Kaikita, K. et al., Circulation 92:1726-1730, 1995; Ikeda, H. et al., Coron. Artery Dis. 5:515-518, 1994). The sensitivity and specificity of membrane-bound P-selectin versus soluble P-selectin for AMI is 71 % versus 76% and 32% versus 45% (Hollander, J.E. et al., J. Am. Coll. Cardiol.,34:95-105,1999). The sensitivity and specificity of mcinbrane-bound P-selectin versus soluble P-selectin for unstable angina + AMI is 71 % versus 79% and 30% versus 35% (Hollander, J.E. et al., J. Am. Coll. Cardiol. 34:95-105, 1999). P-selectin expression is greater in coronary atherectomy specimens from individuals with unstable angina than stable angina (Tenaglia, A.N. et al., Am. J. Cardiol. 79:742-747, 1997). Furthermore, plasma soluble P-selectin may be elevated to a greater degree in patients with AMI than in patients with unstable angina. Plasma soluble and membrane-bound P-selectin also is elevated in individuals with non-insulin dependent diabetes mellitus and congestive heart failure (Nomura, S. et al., Thromb. Haemost. 80:388-392, 1998; O'Connor, C.M. et al., Am. J. Cardiol. 83:1345-1349, 1999): Soluble P-selectin concentration is elevated in the plasma of individuals with idiopathic thrombocytopenic purpura, rheumatoid arthritis, hypercholesterolernia, acute stroke, atherosclerosis, hypertension, acute lung injury, connective tissue disease, thrombotic thrombocytopenic purpura, hemolytic uremic syndrome, disseminated intravascular coagulation, and chronic renal failure (Katayama, M. et al., Br. J. Haematol. 84:702-710, 1993; Haznedaroglu, I.C. et al., Acta Haematol. 101:16-20, 1999; Ertenli, I. et al., J. Rheumatol. 25:1054-1058,1998; Davi, G. et al., Circulation 97:953-957, 1998; Frijns, C.J. et al., Stroke 28:2214-2218, 1997; Blann, A.D. et al., Thromb. Haemost. 77:1077-1080, 1997; Blann, A.D. et al., J. Hum. Hypertens.11:607-609, 1997; Sakamaki, F. et al., A. J. Respir. Crit. Care Med. 151: 1821-1826, 1995; Takeda, I. et al., Int. Arch. Allergy Immunol. 105:128-134, 1994; Chong, B.H. et al., Blood 83:1535-1541, 1994; Bonomini, M. et al., Nephron 79:399-407, 1998). Additionally, any condition that involves platelet activation can potentially be a source of plasma elevations in P-selectin. P-selectin is rapidly presented on the cell surface following platelet of endothelial cell activation. Soluble P-selectin that has been translated from an alternative mRNA lacking a transmembrane domain is also released into the extracellular space following this activation. Soluble P-selectin can also be formed by proteolysis involving membrane-bound P-selectin, either directly or indirectly. Plasma soluble P-selectin is elevated on admission in patients with AMI treated with tPA or coronary angioplasty, with a peak elevation occurring 4 hours after onset (Shimomura, H. et al., Am. J. Cardiol. 81:3197-400,1998). Plasma soluble P-selectin was elevated less than one hour following an anginal attack in patients with unstable angina, and the concentration decreased with time, approaching baseline more than 5 hours after attack onset (Ikeda, H. et al., Circulation 92:1693-1696Y 1995). The plasma concentration of soluble P-selectin can approach 1 µg/ml in ACS (Ikeda, H. et al., Coron. Artery Dis. 5:515-518, 1994). Further investigation into the release of soluble P-selectin into and its removal from the bloodstream need to be conducted. P-selectin may be a sensitive and specific marker of platelet and endothelial cell activation, conditions that support thrombus formation and inflammation. It is not, however, a specific marker of ACS. When used with another marker that is specific for cardiac tissue injury, P-selectin may be useful in the discrimination of unstable angina and AMI from stable angina. Furthermore, soluble Pselectin may be elevated to a greater degree in AMI than in unstable angina. P-selectin normally exists in two forms, mernbrane-bound and soluble. Published investigations note that a soluble form of P-selectin is produced by platelets and endothelial cells, and by shedding of membrane-bound P-selectin, potentially through a proteolytic mechanism. Soluble P-selectin may prove to be the most useful currently identified marker of platelet activation, since its plasma concentration may not be as influenced by the blood sampling procedure as other markers of platelet activation, such as PF4 and β-TG.

Thrombin is a 37 kDa serine proteinase that proteolytically cleaves fibrinogen to form fibrin, which is ultimately integrated into a crosslinked network during clot formation. Antithrombin III (ATIII) is a 65 kDa serine proteinase inhibitor that is a physiological regulator of thrombin, factor XIa, factor Xlla, and factor IXa proteolytic activity. The inhibitory activity of ATIII is dependent upon the binding of heparin. Heparin enhances the inhibitory activity of ATIII by 2-3 orders of magnitude, resulting in almost instantaneous inactivation of proteinases inhibited by ATIII. ATIII inhibits its target proteinases through the formation of a covalent 1:1 stoichiometric complex. The normal plasma. concentration of the approximately 100 kDa thrombin-ATIII complex (TAT) is < 5 ng/ml (50 pM). TAT concentration is elevated in patients with AMI and unstable angina, especially during spontaneous ischemic episodes (Biasucci, L.M. et al., Am. J. Cardiol. 77:85-87, 1996; Kienast, S. et al., Thromb. Haemost. 70:550-553, 1993). Furthermore, TAT maybe elevated in the plasma of individuals with stable angina (Manten, A. et al., Cardiovasc. Res. 40:389-395, 1998). Other published reports have found no significant differences in the concentration of TAT in the plasma of patients with ACS (Manten, A. et al., Cardiovasc. Res. 40:389- 395, 1998; Hoffmeister, H.M. et al., Atheroselerosis 144:151-157, 1999). Further investigation is needed to determine plasma TAT concentration changes associated with ACS. Elevation of the plasma TAT concentration is associated with any condition associated with coagulation activation, including stroke, surgery, trauma, disseminated intravascular coagulation, and thrombotic thrombocytopenic purpura. TAT is formed immediately following thrombin activation in the presence of heparin, which is the limiting factor in this interaction. TAT has a half-life of approximately 5 minutes in the bloodstream (Biasucci, L.M. et al., Am. J. Cardiol. 77:85-87, 1996). TAT concentration is elevated in, exhibits a sharp drop after 15 minutes, and returns to baseline less than 1 hour following coagulation activation. The plasma concentration of TAT can approach 50 ng/ml in ACS (Biasucci, L.M. et al., Circulation 93:212 1 -2127, 1996). TAT is a specific marker of coagulation activation, specifically, thrombin activation. TAT may be useful as a marker of coagulation activation on a diagnostic panel with other markers that are specific for plaque rupture and/or cardiac tissue injury.

D-dimer is a crosslinked fibrin degradation product with an approximate molecular mass of 200 kDa. The normal plasma concentration of D-dimer is < 150 ng/ml (750 pM). The plasma concentration of D-dimer is elevated in patients with AMI and unstable angina, but not stable angina (Hofmneister, H.M. et al., Circulation 91:2520-2527, 1995; Bayes-Genis, A. et al., Thromb. Haemost. 81:865-868, 1999; Gurfinkel, E. et al., Br. Heart J. 71:151-155, 1994; Kruskal, J.B. et al., N. Engl. J. Med. 317: 1361-1365, 1987; Tanaka, M. and Suzuki, A., Thromb. Res. 76:289-298, 1994). The plasma concentration of D-dimer also will be elevated during any condition associated with coagulation and fibrinolysis activation, including stroke, surgery, atherosclerosis, trauma, and thrombotic thrombocytopenic purpura. D-dimer is released into the bloodstream immediately following proteolytic clot dissolution by plasmin. Plasma D-dimer concentrations are elevated soon after ACS onset (within 6 hours), and will remain elevated in proportion to the degree of hypercoagulability of the individual. In this regard, further investigation is needed to determine the kinetics of D-dimer removal form the bloodstream following ACS. The plasma concentration of D-dimer can exceed 2 µg/ml in patients with unstable angina (Gurfinkel, E. et al., Br. Heart J 71:151-155, 1994). Plasma D-dimer is a specific marker of fibrinolysis and indicates the presence of a prothrombotic state associated with AMI and unstable angina. D-dimer is not specific for ACS, and plasma elevations of D-dimer may be associated with various risk factors for ACS. However, when used as a member of a panel that contains markers specific for cardiac injury, D-dimer may allow that discrimination of unstable angina and AMI from stable angina. This differentiation may allow physicians to more effectively treat patients presenting with, acute chest pain.

von Willebrand factor (vWF) is a plasma protein produced by platelets, megakaryocytes, and endothelial cells composed of 220 kDa monomers that associate to form a series of high molecular weight multimers. These multimers normally range in molecular weight from 600-20,000 kDa. vWF participates in the coagulation process by stabilizing circulating coagulation factor VIII and by mediating platelet adhesion to exposed subendothelium, as well as to other platelets. The A1 domain of vWF binds to the platelet glycoprotein Ib-IX-V complex and non-fibrillar collagen type VI, and the A3 domain binds fibrillar collagen types I and III (Emsley, J. et al., J. Biol. Chem. 273:10396-10401, 1998). Other domains present in the vWF molecule include the integrin binding domain, which mediates platelet-platelet interactions, the the protease cleavage domain, which appears to be relevant to the pathogenesis of type 11A von Willebrand disease. The interaction of vWF with platelets is tightly regulated to avoid interactions between vWF and platelets in normal physiologic conditions. vWF normally exists in a globular state, and it undergoes a conformation transition to an extended chain structure under conditions of high sheer stress, commonly found at sites of vascular injury. This conformational change exposes intramolecular domains of the molecule and allows vWF to interact with platelets. Furthermore, shear stress may cause vWF release from endothelial cells, making a larger number of vWF molecules available for interactions with platelets. The conformational change in vWF can be induced in vitro by the addition of non-physiological modulators like ristocetin and botrocetin (Miyata, S. et al., J. Biol. Chem. 271:9046-9053, 1996). At sites of vascular injury, vWF rapidly associates with collagen in the subendothelial matrix, and virtually irreversibly binds platelets, effectively forming a bridge between platelets and the vascular subendothelium at the site of injury. Evidence also suggests that a conformational change in vWF may not be required for its interaction with the subendothelial matrix (Sixma, J.J. and de Groot, P.G., Mayo Clin. Proc. 66:628-633, 1991). This suggests that vWF may bind to the exposed subendothelial matrix at sites of vascular injury, undergo a conformational change because of the high localized shear stress, and rapidly bind circulating platelets, which will be integrated into the newly formed thrombus. Measurement of the total amount of YWF would allow one who is skilled in the art to identify changes in total vWF concentration associated with stroke or cardiovascular disease. This measurement could be performed through the measurement of various forms of the vWF molecule. Measurement of the A1 domain would allow the measurement of active vWF in the circulation, indicating that a procoagulant state exists because the A1 domain is accessible for platelet binding. In this regard, an assay that specifically measures vWF molecules with both the exposed A1 domain and either the integrin binding domain or the A3 domain would also allow for the identification of active vWF that would be available for mediating platelet-platelet interactions or mediate crosslinking of platelets to vascular subendothelium, respectively. Measurement of any of these vWF forms, when used in an assay that employs antibodies specific for the protease cleavage domain may allow assays to be used to determine the circulating concentration of various vWF forms in any individual, regardless of the presence of von Willebrand disease. The normal plasma concentration of vWF is 5-10 µg/ml, or 60-110% activity, as measured by platelet aggregation. The measurement of specific forms of vWF may be of importance in any type of vascular disease, including stroke and cardiovascular disease. The plasma vWF concentration is reportedly elevated in individuals with AMI and unstable angina, but not stable angina (Goto, S. et al., Circulation 99:608-613, 1999; Tousoulis, D. et al., Int. J. Cardiol, 56:259-262, 1996; Yazdani, S. et al., J Am Coll Cardiol 30:1284-1287, 1997; Montalescot, G. et al., Circulation 98:294-299). Furthermore, elevations of the plasma vWF concentration may be a predictor of adverse clinical outcome in patients with unstable angina (Montalescot, G. et al., Circulation 98:294-299). vWF concentrations also have been demonstrated to be elevated in patients with stroke and subarachnoid hemorrhage, and also appear to be useful in assessing risk of mortality following stroke (Blann, A. et al., Blood Coagul. Fibrinolysis 10:277-284,1999; Hirashima, Y. et al.. Neurochem Res. 22:1249-1255, 1997; Catto, A.J. et al., Thromb. Hemost. 77:1104-1108, 1997). The plasma concentration of vWF may be elevated in conjunction with any event that is associated with endothelial cell damage or platelet activation. vWF is present at high concentration in the bloodstream, and it is released from platelets and endothelial cells upon activation. vWF would likely have the greatest utility as a marker of platelet activation or, specifically, conditions that favor platelet activation and adhesion to sites of vascular injury. The conformation of vWF is also known to be altered by high shear stress, as would be associated with a partially stenosed blood vessel. As the blood flows past a stenosed vessel, it is subjected to shear stress considerably higher than what it encounters in the circulation of an undiseased individual. Another aspect of this invention measures the forms of vWF that arise from shear stress and the correlation of the forms to the presence of ACS.

Tissue factor (TF) is a 45 kDa cell surface protein expressed in brain, kidney, and heart, and in a transcriptionally regulated manner on perivascular cells and monocytes. TF forms a complex with factor VIIa in the presence of Ca²⁺ ions, and it is physiologically active when it is membrane bound. This complex proteolytically cleaves factor X to form factor Xa. It is normally sequestered from the bloodstream. Tissue factor can be detected in the bloodstream in a soluble form, bound to factor VIIa, or in a complex with factor VIIa, and tissue factor pathway inhibitor that can also include factor Xa. TF also is expressed on the surface of macrophages, which are commonly found in atheroselerotic plaques. The normal serum concentration of TF is < 0.2 ng/ml (4.5 pM). The plasma TF concentration is elevated in patients with ischemic heart disease (Falciani, M. et al., Thromb. Haemost. 79:495-499, 1998). TF is elevated in patients with unstable angina and AMI, but not in patients with stable anclina (Falciani, M. et al., Thromb. Haemost. 79:495-499, 1998; Suefuji, H. et al., Am. Heart J. 134:253-259, 1997; Misumi, K. et al., Am. J. Cardiol. 81:22-26,1998). Furthermore, TF expression on macrophages and TF activity in atherosclerotic plaques is more common in unstable angina than stable angina (Soejima, H. et al., Circulation 99:2908-2913, 1999; Kaikita, K. et al., Arterioscler. Thromb. Vasc, Biol. 17:2232-2237, 1997; Ardissino, D. et al., Lancet 349:769-771, 1997). The differences in plasma TF concentration in stable versus unstable angina may not be of statistical significance. Elevations in the serum concentration of TF are associated with any condition that causes or is a result of coagulation activation through the extrinsic pathway. These conditions can include subarachnoid hemorrhage, disseminated intravascular coagulation, renal failure, vasculitis, and sickle cell disease (Hirashima, Y. et al., Stroke 28:1666-1670, 1997; Takahashi, H. et al., Am. J. Hematol, 46:333-337, 1994; Koyama, T. et al., Br. J. Haematol. 87:343-347, 1994). TF is released immediately when vascular injury is coupled with extravascular cell injury. TF levels in ischemic heart disease patients can exceed 800 pg/ml within 2 days of onset (Falciani, M. et al., Thromb. Haemost. 79:495-499, 1998. TF levels were decreased in the chronic phase of AMI, as compared with the chronic phase (Suefuji, H. et al., Am. Heart J. 134:253 -259, 1997). TF is a specific marker for activation of the extrinsic coagulation pathway and the presence of a general hypercoagulable state. It may be a sensitive marker of vascular injury resulting from plaque rupture and could have some benefit as a member of a panel. It is not specific for ACS, can be elevated in many disease states, and may even be artificially elevated by the blood sampling procedure. However, it may be possible to use TF as a marker to rule out patients for thrombolytic therapy. The infusion of tissue-type plasminogen activator (tPA) during thrombolytic therapy results in an activation of fibrinolysis, and the patient is unable to maintain blood clots. The administration of tPA to an individual with vascular injury could ultimately result in hemorrhage.

The coagulation cascade can be activated through either the extrinsic or intrinsic pathways. These enzymatic pathways share one final common pathway. The first step of the common pathway involves the proteolytic cleavage of prothrombin by the factor Xa/factor Va prothrombinase complex to yield active thrombin. Thrombin is a serine proteinase that proteolytically cleaves fibrinogen. Thrombin first removes fibrinopeptide A from fibrinogen, yielding desAA fibrin monomer, which can form complexes with all other fibrinogen-derived proteins, including fibrin degradation products, fibrinogen degradation products, desAA fibrin, and fibrinogen. The desAA fibrin monomer is generically referred to as soluble fibrin, as it is the first product of fibrinogen cleavage, but it is not yet crosslinked via factor XIIIa into an insoluble fibrin clot. DesAA fibrin monomer also can undergo further proteolytic cleavage by thrombin to remove fibrinopeptide B, yielding desAABB fibrin monomer. This monomer can polymerize with other desAABB fibrin monomers to form soluble desAABB fibrin polymer, also referred to as soluble fibrin or thrombus precursor protein (TpP™). TpP™ is the immediate precursor to insoluble fibrin, which forms a "mesh-like" structure to provide structural rigidity to the newly formed thrombus. In this regard, measurement of TpP™ in plasma is a direct measurement of active clot formation. The normal plasma concentration of TpP™ is < 6 ng/ml (Laurino, J.P. et al., Ann. Clin. Lab. Sci. 27:338-345, 1997). American Biogenetic Sciences has developed an assay for TpP™ (US Patent Nos. 5453359 and 5843690) and states that its TpP™ assay can assist in the early diagnosis of AMI, the ruling out of AMI in chest pain patients, and the identification of patients with unstable angina that will progress to AMI. Other studies have confirmed that TpP™ is elevated in patients with AMI, most often within 6 hours of onset (Laurino, J.P. et al., Ann. Clin. Lab. Sci. 27:338-345, 1997, Carville, D.G. et al., Clin. Chem. 42:1537-1541, 1996). The plasma concentration of TpP™ is also elevated in patients with unstable angina, but these elevations may be indicative of the severity of angina and the eventual progression to AMI (Laurino, J.P. et al., Ann. Clin. Lab. Sci. 27:338-345, 1997). The concentration of TpP™ in plasma will theoretically be elevated during any condition that causes or is a result of coagulation activation, including disseminated intravascular coagulation, deep venous thrombosis, congestive heart failure, surgery, cancer, gastroenteritis, and cocaine overdose (Laurino, J.P. et al., Ann. Clin. Lab. Sci. 27:338-345, 1997). TpP™ is released into the bloodstream immediately following thrombin activation. TpP™ likely has a short half-life in the bloodstream because it will be rapidly converted to insoluble fibrin at the site of clot formation. Plasma TpP™ concentrations peak within 3 hours of AMI onset, returning to normal after 12 hours from onset. The plasma concentration of TpP™ can exceed 30 ng/ml in CVD (Laurino, J.P. et al., Ann. Clin. Lab. Sci. 27:338-345, 1997). TpP™ is a sensitive and specific marker of coagulation activation. It has been demonstrated that TpP™ is useful in the diagnosis of AMI, but only when it is used in conjunction with a specific marker of cardiac tissue injury. TpP™ is not a specific marker of ACS, and its concentration will be elevated in numerous disease states that involve coagulation activation, including conditions that are considered risk factors for the development of ACS. TpP™ may also be useful in determining the severity of unstable angina. American Biogenetic Sciences, Inc. instructs users of the TpP™ ELISA assay kit to collect blood using citrate as an anticoagulant, and they recommend against using EDTA. The effect of the anticoagulant used during blood sampling on plasma TpP™ levels is currently unclear. If the blood sampling procedure can be controlled, TpP™ may be the best available marker for coagulation activation.

Further, markers of group (i) are preferably used according to the invention, which are acute markers as well as specific markers for myocardial injury. Markers of this type are associated with acute coronary disease (ACS) and indicate, for example, myocardial injury and acute myocardial infarction (AMI). Examples of said markers are anexin V, also called lipocortin V, endonexin II, calphobindin I, calcium binding protein 33, placental anticoagulant protein I, thromboplastin inhibitor, vascular anticoagulant-α, anchorin CII, B-type natriuretic peptide (BNP), also called brain-type natriuretic peptide, enolase, TnT, Tnl, fTnT, CK, GP, H-FABP, PG AM as well as S-100.

Annexin V, also called lipocortin V, endonexin II, calphobindin I, calcium binding protein 33, placental anticoagulant protein I, thromboplastin inhibitor, vascular anticoagulant-a, and anchorin CII, is a 33 kDa calcium-binding protein that is an indirect inhibitor and regulator of tissue factor. Annexin V is composed of four homologous repeats with a consensus sequence common to all annexin family members, binds calcium and phosphatidyl serine, and is expressed in a wide variety of tissues, including heart, skeletal muscle, liver, and endothelial cells (Giambanco, I. et al., J. Histochem. Cytochem. 39:P1189-1198, 1991; Doubell, A.F. et al., Cardiovasc. Res. 27:1359-1367, 1993). The normal plasma concentration of annexin V is < 2 ng/ml (Kaneko, N. et al., Clin. Chim. Acta 251:65-80, 1996). The plasma concentration of annexin V is elevated in individuals with AMI (Kaneko, N. et al., Clin. Chim. Acta 1-51:65-80, 1996). Due to its wide tissue distribution, elevation of the plasma concentration of annexin V may be associated with any condition involving noncardiac tissue injury. However, one study has found that plasma annexin V concentrations were not significantly elevated in patients with old myocardial infarction, chest pain syndrome, valvular heart disease, lung disease, and kidney disease (Kaneko, N. et al., Clin. Chim. Acta 251:65-80, 1996). These previous results require confirmation before the clinical utility of annexin V as an ACS marker can be determined. Annexin V is released into the bloodstream soon after AMI onset. The annexin V concentration in the plasma of AMI patients decreased from initial (admission) values, suggesting that it is rapidly cleared from the bloodstream (Kaneko, N. et al., Clin. Chim. Acta 251:65-80, 1996).

B-type natriuretie peptide (BNP), also called brain-type natriuretic peptide is a 32 amino acid, 4 kDa peptide that is involved in the natriuresis system to regulate blood pressure and fluid balance (Bonow, R.O., Circulation 93:1946-1950, 1996). The precursor to BNP is synthesized as a 108-amino acid molecule, referred to as "pre pro BNP" that is proteolytically processed into a 76-amino acid N-terminal peptide (amino acids 1-76), referred to as "NT pro BNP" and the 32-amino acid mature hormone, referred to as BNP or BNP 32 (amino acids 77-108). It has been suggested that each of these species - NT pro-BNP, BNP-32, and the pre pro BNP - can circulate in human plasma (Tateyama et al., Biochem. Biophys. Res. Commun. 185: 760-7 (1992); Hunt et al., Biochem. Biophys. Res. Commun. 214: 1175-83 (1995)). The 2 forms, pre pro BNP and NT pro BNP, and peptides which are derived from BNP, pre pro BNP and NT pro BNP and which are present in the blood as a result of proteolyses of BNP, NT pro BN-P and pre pro BNP, are collectively described as markers related to or associated with BNP. Proteolytic degradation of BNP and of peptides related to BNP have also been described in the literature and these proteolytic fragments are also encompassed it the term "BNP related peptides". BNP and BNP-related peptides are predominantly found in the secretory granules of the cardiac ventricles, and are released from the heart in response to both ventricular volume expansion and pressure overload (Wilkins, M. et al., Lancet 349:1307-1310, 1997). Elevations of BNP are associated with raised atrial and pulmonary wedge pressures, reduced ventricular systolic and diastolic function, left ventricular hypertrophy, and myocardial infarction (Sagnella, G.A, Clinical Science 95:519-529, 1998). Furthermore, there are numerous reports of elevated BNP concentration associated with congestive heart failure and renal failure. While BNP and BNP-related peptides are likely not specific for ACS, they may be sensitive markers of ACS because they may indicate not only cellular damage due to ischemia, but also a perturbation of the natriuretic system associated with ACS. The term "BNP" as used herein refers to the mature 32-amino acid BNP molecule itself. As the skilled artisan will recognize, however, other markers related to BNP may also serve as diagnostic or prognostic indicators in patients with ACS. For example, BNP is synthesized as a 108-amino acid pre pro-BNP molecule that is proteolytically processed into a 76-amino acid "NT pro BNP" and the 32-ammio acid BNP molecule. Because of its relationship to BNP, the concentration of NT pro-BNP molecule can also provide diagnostic or prognostic information in patients. The phrase "marker related to BNP or BNP related peptide" refers to any polypeptide that originates from the pre pro-BNP molecule, other than the 32-amino acid BNP molecule itself. Thus, a marker related to or associated with BNP includes the NT pro-BNP molecule, the pro domain, a fragment of BNP that is smaller than the entire 32-amino acid sequence, a fragment of pre pro-BN-P other than BNP, and a fragment of the pro domain. One skilled in the art will also recognize that the circulation contains proteases which can proteolyze BNP and BNP related molecules and that these proteolyzed molecules (peptides) are also considered to be "BNP related" and are additionally subjects of this invention.

Enolase is a 78 kDa homo- or heterodimeric cytosolic protein produced from α, β, and γ subunits. Enolase catalyzes the interconversion of 2-phosphoglycerate and phosphoenolpyruvate in the glycolytic pathway. Enolase is present as αα, αβ, ββ, αγ, and γγ isoforms. The α subunit is found in most tissues, the β subunit is found in cardiac and skeletal muscle, and the γ subunit is found primarily in neuronal and neuroendocrine tissues. β-enolase is composed of αβ and ββ enolase, and is specific for muscle. The normal plasma concentration of β-enolase is < 10 ng/ml (120 pM). ßenolase is elevated in the serum of individuals with AMI, but not in individuals with angina (Nomura, M. et al., Br. Heart J. 58:29-33, 1987; Herraez-Dominguez, M.V. et al., Clin. Chim. Acta 64:307-315, 1975). Further investigations into possible changes in plasma β-enolase concentration associated with unstable and stable angina need to be performed. The plasma concentration of β-enolase is elevated during heart surgery, muscular dystrophy, and skeletal muscle injury (Usui, A. et aL, Cardiovasc. Res. 23:737-740, 1989; Kato, K. et al., Clin. Chim. Acta 131:75-85, 1983; Matsuda, H. et al., Forensic Sci. Int. 99:197-208, 1999). β-enolase is released into the bloodstream immediately following cardiac or skeletal muscle injury. The plasma β-enolase concentration was elevated to more than 150 ng/ml in the perioperative stage of cardiac surgery, and remained elevated for 1 week. Serum β-enolase concentrations peaked approximately 12-14 hours after the onset of chest pain and AMI and approached baseline after 1 week had elapsed from onset, with maximum levels approaching 1 µg/ml (Kato, K. et al., Clin. Chim. Acia 131:75-85, 1983; Nomura, M. et al., Br. Heart J. 58:29-33, 1987).

Troponin I (Tnl) is a 25 kDa inhibitory element of the troponin complex, found in all striated muscle tissue. Tnl binds to actin in the absence of Ca²⁺, inhibiting the ATPase activity of actomyosin. A Tnl isoform, that is found in cardiac tissue (cTnl) is 40% divergent from skeletal muscle Tnl, allowing both isoforms to be immunologically distinguished. The normal plasma concentration of cTnl is < 0.1 ng/ml (4 pM). The plasma cTnl concentration is elevated in patients with AMI. Investigations into changes in the plasma cTnl concentration in patients with unstable angina have yielded mixed results, but cTnl is not elevated in the plasma of individuals with stable angina (Benamer, H. et al., Am. J. Cardiol. 82:845-850, 1998; Bertinchant, J.P. et al., Clin. Biochem. 29:587-594,1996, Tanasijevic, M.S. et al., Clin. Cardiol. 22:13-16, 1999: Musso, P. et al., J. Ital. Cardiol. 26:1013-1023, 1996; Holvoet, P. et al., JAMA 281:1718-1721, 1999; Holvoet, P. et al., Circulation 98:1487-1494, 1998). The mixed results associated with unstable angina suggest that cTnl may be useful in determining the severity of unstable angina because the extent of myocardial ischemia is directly proportional to unstable angina severity. The plasma cTnl concentration may be elevated in conjunction with cardiac trauma, congestive heart failure, and cardiac surgery, non-ischem:ic dilated cardiomyopathy, muscular disorders, CNS disorders, HIV infection, chronic renal failure, sepsis, lung disease, and endocrine disorders (Khan, I.A. et al., Am. J. Emerg. Med. 17:225-229, 1999). This apparent nonspecificity may be related to the quality and specificity of the antibodies used in the immunoassay. cTnl is released into the bloodstream following cardiac cell death. The plasma concentration of cTnl in patients with AMI is significantly elevated 4-6 hours after onset, peaks between 12-16 hours, and can remain elevated for one week. The release kinetics of cTnl associated with unstable angina may be similar. The measurement of specific forms of cardiac troponin, including free cardiac troponin I and complexes of cardiac troponin I with troponin C and/or T may provide the user with the ability to identify various stages of ACS.
Free and complexed cardiac-troponin T may be used in a manner analogous to that described above for cardiac troponin I. Cardiac troponin T complex may be useful either alone or when expressed as a ratio with total cardiac troponin I to provide information related to the presence of progressing myocardial damage. Ongoing ischemia may result in the release of the cardiac troponin TIC complex, indicating that higher ratios of cardiac troponin TIC:total cardiac troponin I may be indicative of continual damage caused by unresolved ischemia.

Creatine kinase (CK) is a 85 kDa cytosolic enzyme that catalyzes the reversiible formation ADP and phosphocreatine from ATP and creatine. CK is a homo- or heterodimer composed of M and B chains. CK-MB is the isoform that is most specific for cardiac tissue, but it is also present in skeletal muscle and other tissues. The normal plasma concentration of CK-MB is < 5 ng/ml. The plasma CK-MB concentration is significantly elevated in patients with AMI. Plasma CK-MB is not elevated in patients with stable angina, and investigation into plasma CK-MB concentration elevations in patients with unstable angina have yielded mixed results (Thygesen, K. et al., Eur. J Clin. Invest. 16:14, 1986, Koukkunen, H. et al., Ann. Med 30:488-496, 1998, Bertinchant. J.P. et al., Clin. Biochem. 29:587-594,1996; Benamer, H. et al., Am. J. Cardiol. 82:845-850, 1998; Norregaard-Hansen, K. et al., Eur. Heart J. 13:188-193, 1992). The mixed results associated with unstable angina suggest that CKMB may be useful in determining the severity of unstable angina because the extent of myocardial ischemia is directly proportional to unstable angina severity. Elevations of the plasma CK-MB concentration are associated with skeletal muscle injury and renal disease. CK-MB is released into the bloodstream following cardiac cell death. The plasma concentration of CK-MB in patients with AMI is significantly elevated 4-6 hours after onset, peaks between 12-24 hours, and returns to baseline after 3 days. The release kinetics of CK-MB associated with unstable angina may be similar.

Glycogen phosphorylase (GP) is a 188 kDa intracellular allosteric enzyme that catalyzes the removal of glucose (liberated as glucose-1-phosphate) from the nonreducing ends of glycogen in the presence of inorganic phosphate during glycogenolysis. GP is present as a homodimer, which associates with another homodimer to form a tetrameric enzymatically active phosphorylase A. There are three isoforms of GP that can be immunologically distinguished. The BB isoform is found in brain and cardiac tissue, the MM isoform is found in skeletal muscle and cardiac tissue, and the LL isoform is predominantly found in liver (Mair, J. et al., Br. Heart J. 72:125127, 1994). GP-BB is normally associated with the sarcoplasmic reticulum glycogenolysis complex, and this association is dependent upon the metabolic state of the myocardium (Mair, J., Clin. Chim. Acta 272:79-86, 1998). At the onset of hypoxia, glycogen is broken down, and GP-BB is converted from a bound form to a free cytoplasmic form (Krause, E.G. et al.. Mol. Cell Biochem. 160-161:289-295, 1996). The normal plasma GP-BB concentration is < 7 ng/ml (36 pM). The plasma GP-BB concentration is significantly elevated in patients with AMI and unstable angina with transient ST-T elevations, but not stable angina (Mair, J. et al., Br. Heart J. 72:125-127, 1994, Mair, J., Clin. Chim. Acta 272:79-86, 1998; Rabitzsch, G. et al., Clin. Chem. 41:966-978, 1995; Rabitzsch, G. et al., Lancet 341:1032-1033, 1993). Furthermore, GP-BB also can be used to detect perioperative AMI and myocardial ischemia in patients undergoing coronary artery bypass surgery (Rabitzsch, G. et al., Biomed. Biochim. Acta 46:S584-S588, 1987; Mair, P. et al., Eur. J Clin. Chem. Clin. Biochem. 32:543-547, 1994). GP-BB has been demonstrated to be a more sensitive marker of unstable angina and AMI early after onset than CK-MB, cardiac tropopnin T, and myoglobin (Rabitzsch, G. et al., Clin. Chem. 41:966-978, 1995). Because it is also found in the brain, the plasma GP-BB concentration also may be elevated during ischemic cerebral injury. GP-BB is released into the bloodstream under ischernic conditions that also involve an increase in the permeability of the cell membrane, usually a result of cellular necrosis. GP-BB is significantly elevated within 4 hours of chest pain onset in individuals with unstable angina and transient ST-T ECG alterations, and is significantly elevated while myoglobin, CK-MB, and cardiac troponin T are still within normal levels (Mair, J. et al., Br. Heart J. 72:125-127, 1994). Furthermore, GP-BB can be significantly elevated 1-2 hours after chest pain onset in patients with AMI (Rabitzsch, G. et al., Lancet 341:1032-1033, 1993). The plasma GP-BB concentration in patients with unstable angina and AMI can exceed 50 ng/ml (250 pM) (Mair, J. et al., Br. Heart J. 72:125-127, 1994; Mair, J., Clin. Chim. Acta 272:7986, 1998; Krause, E.G. et al., Mol. Cell Biochem. 160-161:289-295, 1996; Rabitzsch, G. et al., Clin. Chem. 41:966-978, 1995; Rabitzsch, G. et al., Lancet 341:1032-1033, 1993). GP-BB appears to be a very sensitive marker of myocardial ischemia, with specificity similar to that of CK-BB. GP-BB plasma concentrations are elevated within the first 4 hours after AIMI onset, which suggests that it may be a very useful early marker of myocardial damage. Furthermore, GP-BB is not only a more specific marker of cardiac tissue damage, but also ischemia, since it is released to an unbound form during cardiac ischemia and would not normally be released upon traumatic injury. This is best illustrated by the usefulness of GP-BB in detecting myocardial ischemia during cardiac surgery. GP-BB may be a very useful marker of early myocardial ischemia during AMI and severe unstable angina.

Heart-type fatty acid binding protein (H-FABP) is a cytosolic 15 kDa lipid-binding protein involved in lipid metabolism. Heart-type FABP antigen is found not only in heart tissue, but also in kidney, skeletal muscle, aorta, adrenals, placenta, and brain (Veerkamp, J.H. and Maatman, R.G., Prog. Lipid Res. 34:17-52, 1995; Yoshimoto, K. et al., Heart Vessels 10:304-309, 1995). Furthermore, heart-type FABP mRNA can be found in testes, ovary, lung, mammary gland, and stomach (Veerkamp, J. H. and Maatman, R. G, Prog. Lipid Res. 34:17-52,1995). The normal plasma concentration of FABP is < 6 ng/ml (400 pM). The plasma H-FABP concentration is elevated in patients with AMI and unstable angina (Ishii, J. et al., Clin. Chem. 43:1372-1378, 1997; Tsuji, R. et al., Int. J. Cardiol. 41:209-217, 1993). Furthermore, H-FABP may be useful in estimating infarct size in patients with AMI (Glatz, J. F. et al., Br. Heart J. 71:135-140 1994). Myocardial tissue as a source of H-FABP can be confirmed by determinmig the ratio of myoglobin/FABP (grams/grams), A ratio of approximately 5 indicates that FABP is of myocardial origin, while a higher ratio indicates skeletal muscle sources (Van Nieuwenhoven, F.A. et al., Circulation 92:28482854- 1995). Because of the presence of H-FABP in skeletal muscle, kidney and brain, elevations in the plasma H-FABP concentration may be associated with skeletal muscle injury, renal disease, or stroke. H-FABP is released into the bloodstrearn following cardiac tissue necrosis. The plasma H-FABP concentration can be significantly elevated 1-2 hours after the onset of chest pain, earlier than CK-MB and myoglobin (Tsuji, R. et al., Int. J. Cardiol. 41:209-217, 1993; Van Nieuwenhoven, F.A. et al., Circulation 92:2848-2854, 1995; Tanaka, T. et al., Clin, Biochem. 24:195-201, 1991). Additionally, H-FABP is rapidly cleared from the bloodstream, and plasma concentrations return to baseline after 24 hours after AMI onset (Glatz, S.P. et al., Br. Heart J. 71:13 5-140, 1994; Tanaka, T. et al., Clin. Biochem. 24:195-201, 1991).

Phosphoglyceric acid mutase (PGAM) is a 57 kDa homo- or heterodimenic intracellular glycolytic enzyme composed of 29 kDa M or B subunits that catalyzes the interconversion of 3-phosphoglycerate to 2-phosphoglycerate in the presence of magnesium. Cardiac tissue contains isozymes MM, MB, and BB, skeletal muscle contains primarily PGAM-MM, and most other tissues contain PGAM-BB (Durany, N. and Carreras, J., Comp. Biochem. Physiol. B. Biochem. Mol. Biol. 114:217-223, 1996). Thus, PGAM-MB is the most specific isozyme for cardiac tissue. PGAM is elevated in the plasma of patients with AMI but further studies need to be performed to determine changes in the plasma PGAM concentration associated with AMI, unstable angina and stable angina (Mair, J., Crit. Rev. Clin. Lab. Sci. 34:1-66, 1997). Plasma PGAM-MB concentration elevations may be associated with unrelated myocardial or possibly skeletal tissue damage. PGAM-MB is most likely released into the circulation following cellular. necrosis. PGAM has a half-life of less than 2 hours in the bloodstream of rats (Grisolia, S. et al., Physiol. Chem. Phys. 8:37-52, 1976).

S-100 is a 21 kDa homo- or heterodimenic cytosolic, Ca2²⁺-binding protein produced from α and β subunits. It is thought to participate in the activation of cellular processes along the Ca²⁺-dependent signal transduction pathway (Bonfrer, J.M. et al., Br. J. Cancer 77:2210-2214, 1998). S-100ao (αα isoform) is found in striated muscles, heart and kidney, S-100a (αβ isoform) is found in glial cells, but not in Schwann cells, and S -100b (ββ isoform) is found in high concentrations in glial cells and Schwann cells, where it is a major cytosolic component (Kato, K. and Kimura, S., Biochim. Biophys. Acta 842:146-150, 1985; Hasegawa, S. et al., Eur. Urol. 1993). The normal serum concentration of S-100ao is < 0.25 ng/ml (12 pM), and its concentration may be influenced by age and sex, with higher concentrations in males and older individuals (Kikuchi, T. et al., Hinyokika Kiyo 36:1117-1123, 1990; Morita, T. et al., Nippon Hinyokika Gakkai Zasshi 81:1162-1167, 1990; Usui, A. et al., Clin. Chem. 36:639-641, 1990). The serum concentration of S-100ao is elevated in patients with AMI, but not in patients with angina pectoris with suspected AMI (Usui, A. et al.,Clin. Chem. 36:639-641, 1990). Further investigation is needed to determine changes in the plasma concentration of S-100ao associated with unstable and stable angina. Serum S-100ao is elevated in the serum of patients with renal cell carcinoma, bladder tumor, renal failure, and prostate cancer, as well as in patients undergoing open heart surgery (Hasegawa, S. et al., Eur. Urol. 24:393-396, 1993; Kikuchi, T. et aL, Hinyokika Kiyo 36:1117-1123, 1990; Morita, T. et al., Nippon Hinyokika Gakkai Zasshi 81: 11621167, 1990; Usui, A. et al., Clin. Chem. 35:1942-1944, 1989). S-100ao is a cytosolic protein that will be released into the extracellular space following cell death. The serum concentration of S-100ao is significantly elevated on admission in patients with AMI, increases to peak levels 8 hours after admission, decreases and returns to baseline one week later (Usui, A. et al., Clin. Chem. 36:639-641, 1990).

Furthermore, S-100ao appears to be significantly elevated earlier after AMI onset than CK-MB (Usui, A. et al., Clin. Chem. 36:639-641, 1990). The maximum serum S-100ao concentration can exceed 100 ng/ml. S-100ao maybe rapidly cleared from the bloodstream by the kidney, as suggested by the rapid decrease of the serum S-100ao concentration of heart surgery patients following reperfusion and its increased urine concentration, but further investigation is needed to determine the kinetics of S-100ao release into and clearance from the bloodstream in the context of ACS (Usui, A. et al., Clin. Chem. 35:1942-1944, 1989). S-100ao is found in high concentration in cardiac tissue and appears to be a sensitive marker of cardiac injury. Major sources of non-specificity of this marker for ACS include skeletal muscle and renal tissue injury. S-100ao may be significantly elevated soon after AMI onset, and it may allow for the discrimination of AMI from unstable angina. Patients with angina pectoris and suspected AMI, indicating that they were suffering chest pain associated with an ischemic episode, did not have a significantly elevated S-100ao concentration. In spite of its risk of non-specificity, which appears to be no different from that of CK-MB and myoglobin, S-100ao may allow physicians to distinguish AMI from unstable angina.

According to the invention sTfR or/and frataxin or/and ferritin index is preferably used together with at least one of the markers given in groups (i), (ii), (iii) and (iv). Preferably, at least one marker of each of groups (i) to (iv) is used.

In a particularly preferred embodiment, the markers given in group (iv) above are replaced according to the invention by hepcidin, sTfR and frataxin. In another preferred embodiment, the markers of group (iii) are replaced by sTfR and frataxin.

Groups 2, 3 and 4 according to the invention relate to markers which provide information at different stages of a disease. Group 1, for example, refers to the stage of chronic inflammation, group 2 the stage of instable angina and groups 3 and 4 ACS and AMI, respectively.

As a result of that, for example, Epo-iron therapy is the suitable procedure in the case of high sTfR and high hepcidin values and low frataxin values in an early stage. If the stage is unknown, suitable therapy can be chosen by determining group 3 and group 4 markers. For example, if group 3 and group 4 markers are normal, Epo-iron therapy is promising. In the case of suspected ACS and AMI, respectively, a determination of groups 3 and 4 is especially favorable for reliable diagnosis.

According to the invention the coagulation markers given in group (ii) above can be assigned to group 2.

The above-described markers and, in particular, sTfR can be used as independent risk markers in different patient groups. Suitable patient subgroups, for example, are healthy seniors, heart patients as well as diabetes patients.

The above-mentioned risk markers permit an effective therapy and the determination of effective therapies for individual patient groups, respectively. For example, an effective Epo therapy can be indicated at an early stage and be controlled by the above markers.

The simultaneous assessment of the above-mentioned markers as proposed by the invention provides additional prognostic information at different stages of coronary syndromes or/and diabetes mellitus.

Advantageously, further criteria are considered when electing the patients. For example, coronary angiography as well as patients with one stenosis > 30% are inclusion criteria. Exclusion criteria, inter alia, are patients with a surgery or PTCA or oral anticoagulation within the previous 4 weeks. Also excluded are patients suffering from sepsis, a chronic systemic disease (RA), a cancer disease or known renal insufficiency. Patients with a trauma or resuscitation or thrombosis within the previous 12 weeks are preferably excluded as well.

The invention is further illustrated by the attached drawings and the Examples given below.
Fig.1 shows the correlation between sTfR and CAD.
Fig.2 shows Kaplan-Meier curves for sTfR quartiles and cardiovascular end points (myocardial infarction, cardiovascular death, stroke)

### Examples

### Example 1

### Soluble transferrin receptor as novel cardiovascular risk factor

Epidemiological studies dedicated to the clarification of the relationship between body iron and coronary artery disease (CAD) have yielded conflicting results. The soluble transferrin receptor (sTfR) represents a new quantitative assay for evaluation of iron role, but is relationship with CAD has not been explored.

Therefore, a case control study was performed which included 916 consecutive patients (678 cases with angiographically proven CAD (183 females, median age 65.8 years and 229 controls without CAD (135 females, median age 61.1 years). Blood was collected before angiography for determination of sTfR, ferritin and C-reactive protein (CRP).

### Results

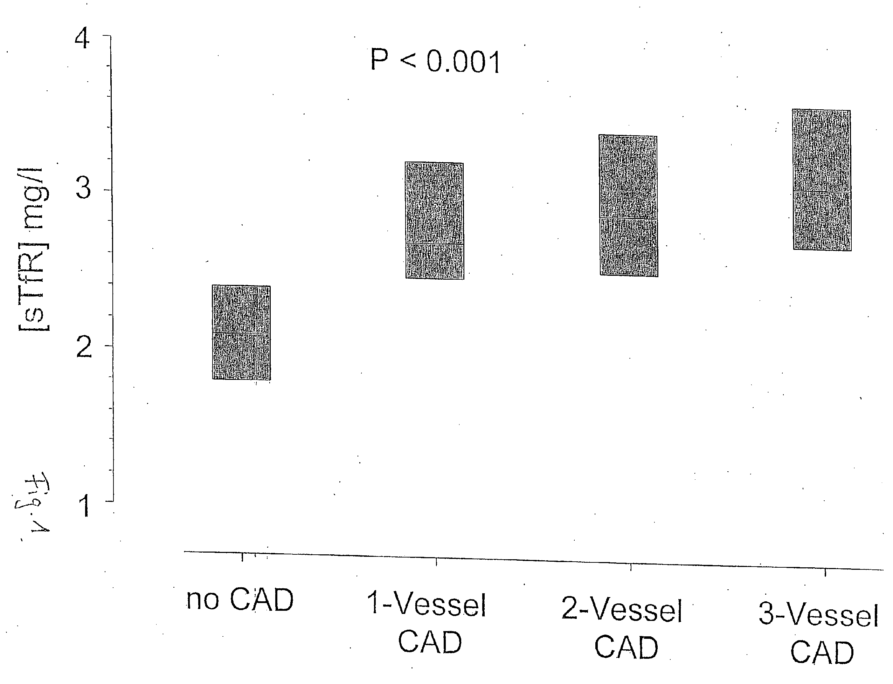

Patients with CAD had higher values (median, 25^{th}-75^{th} percentiles) of sTfR (3.0 mg/L [2.4-3.7] vs 2.1 mg/L [1.7-2.5], CRP (3.7 mg/L [1.4-9.3] vs 1.6 mg/L [0.7-3.9], p<0.001) and ferritin 147.6 ng/ml [77.6-248.8] vs 120.4 ng/ml [74.9-217.6], p=0.08). There was also a correlation between serum values of sTfR and the severity of CAD (see Figure). In multivariate analysis, the sTfR was the strongest independent predictor of CAD (p<0.001) followed by sex (p<0.001), age (p<0.001), hypercholesterolemia (p<0.001), smoking (p<0.001) and CRP (p=0.002). Ferritin was a risk factor for CAD (p=0.78). The results are also shown in Fig.1

### Conclusions

These results strongly support the role of soluble transferrin receptor as a novel risk marker for coronary artery disease. The patients with CAD showed significantly higher values of soluble transferrin receptor (sTfR) than the controls. There was also a correlation between sTfR and the severity of CAD (see also Fig.1). In multivariate analysis the sTfR was the strongest independent predictor of CAD.

### Example 1a

In a study, 892 patients including 664 cases with angiographically proven CAD and 228 controls without CAD were included.

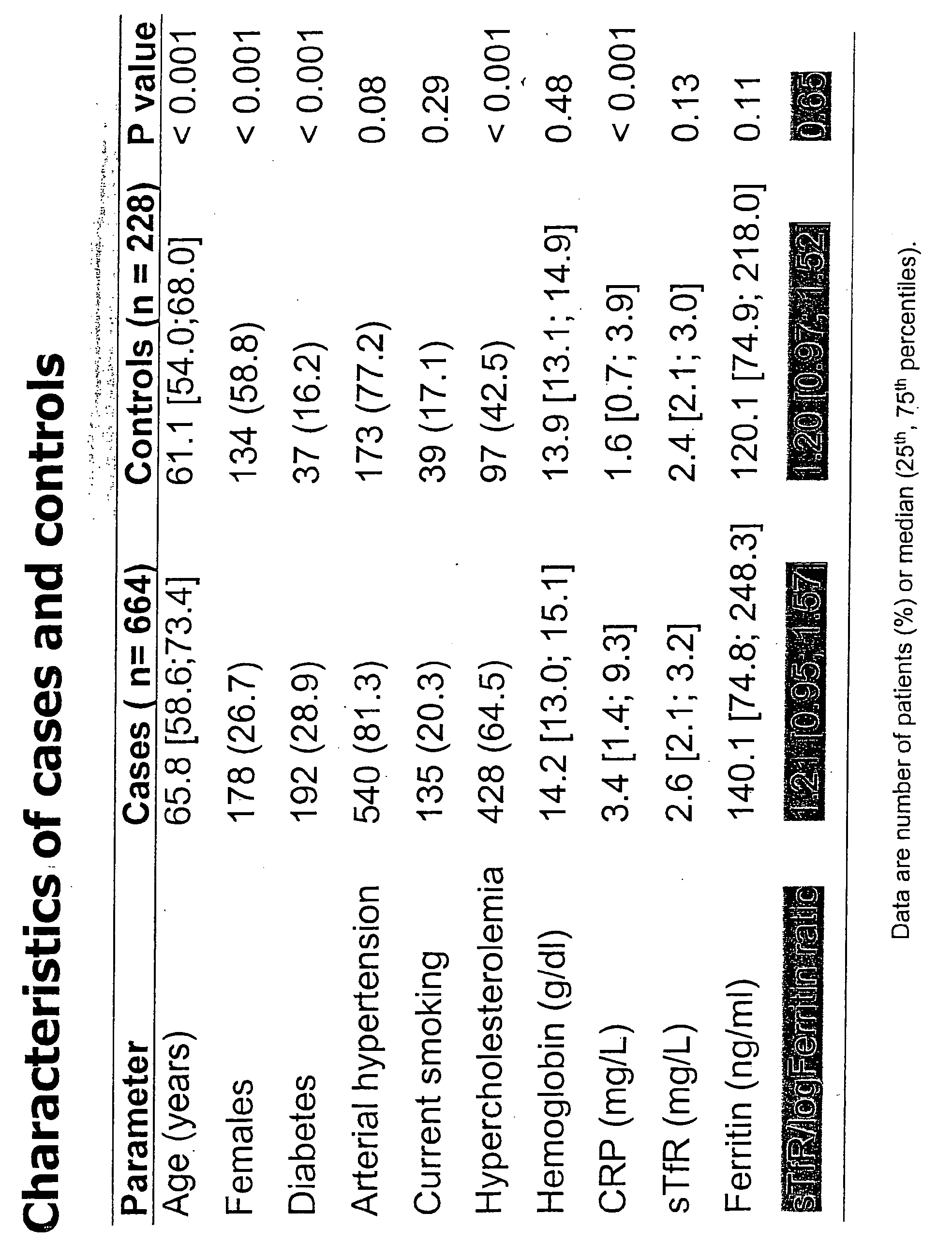

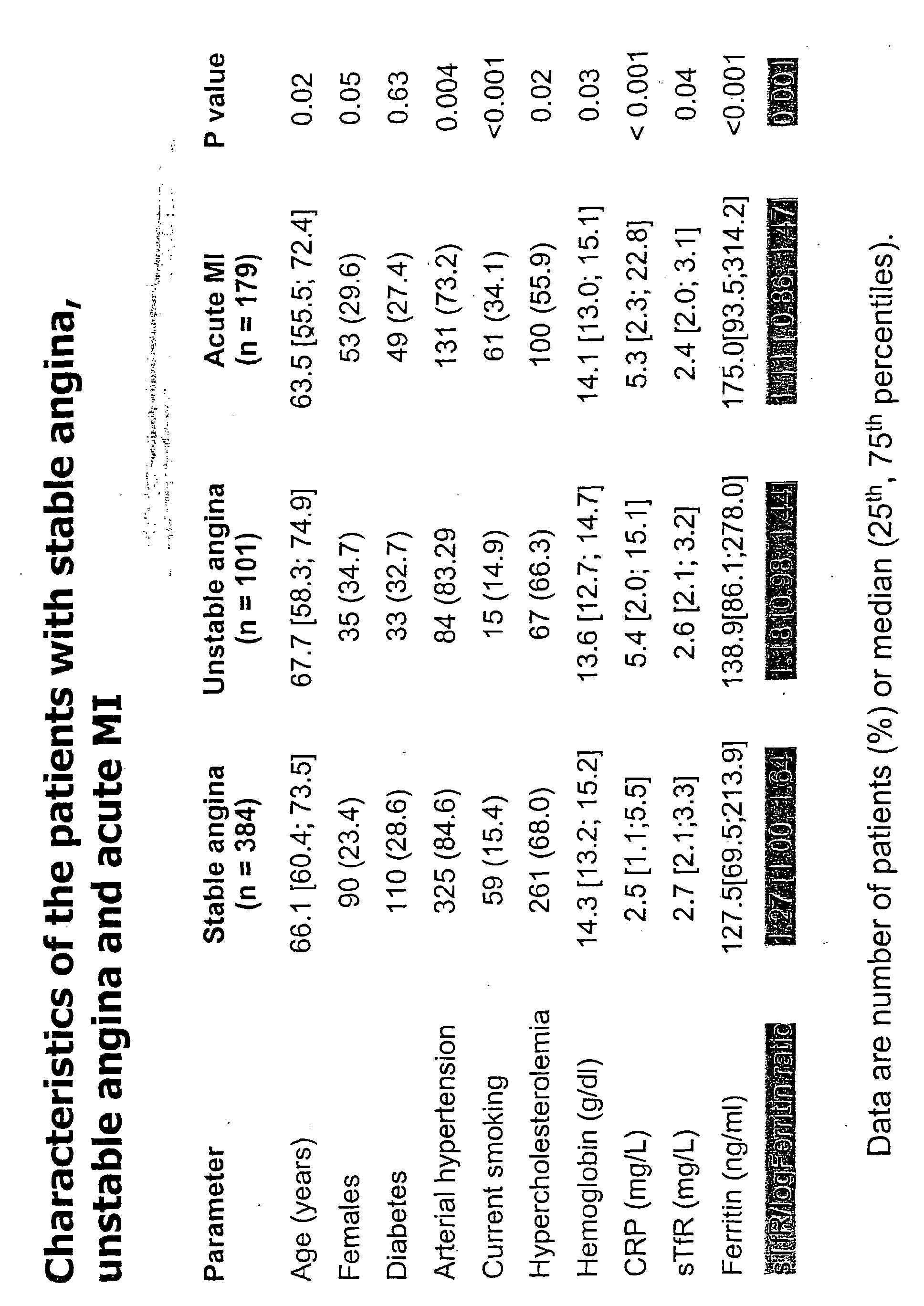

The results were as follows:

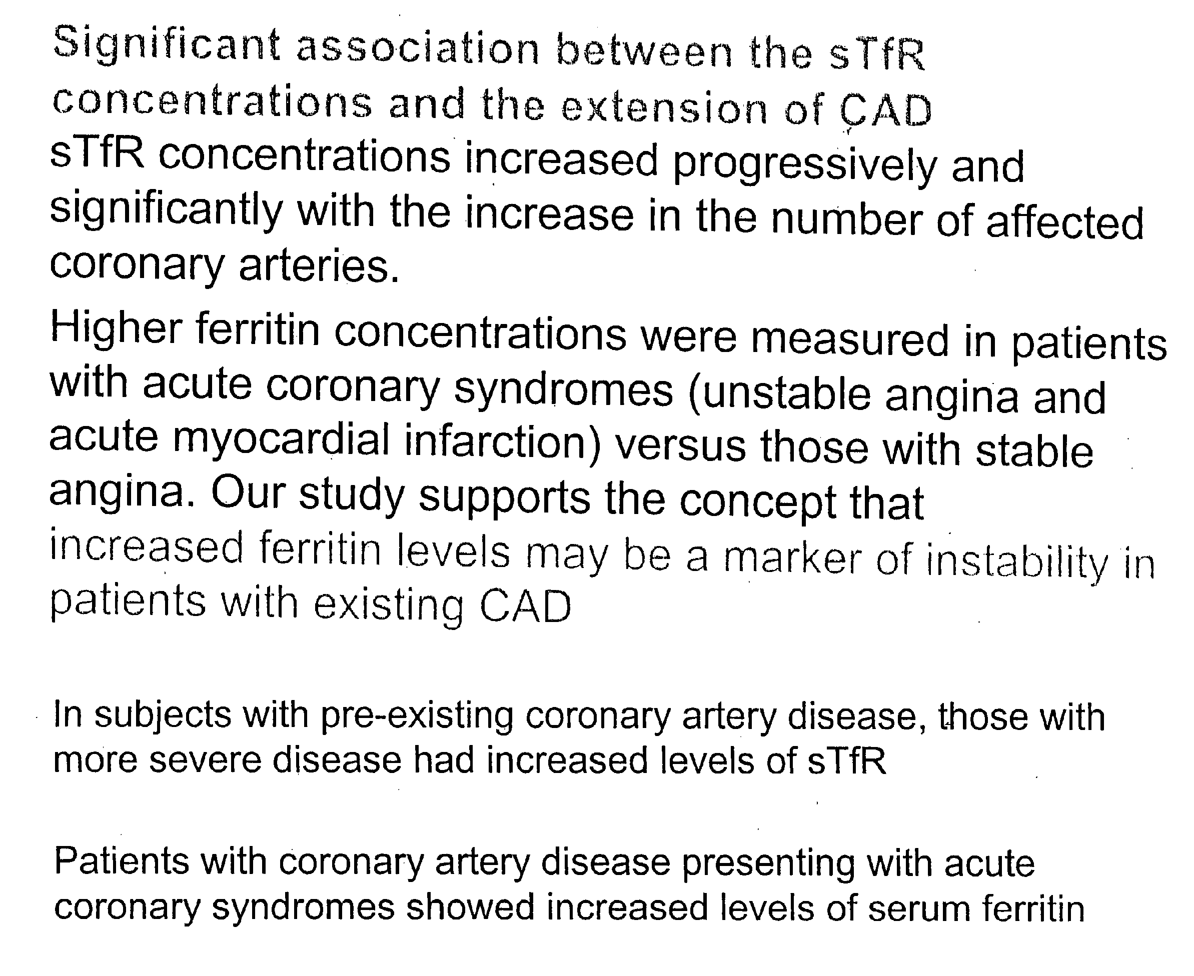

### Example 1b

In a case-control study with 678 patients and 229 controls, patients with CAD had siginficantly higher values of sTfR. There was also a correlation between sTfR and the severity of CAD. sTfR was a strong independent predictor of CAD:

For sTfR (mg/l) the following values were determined in different patient groups:

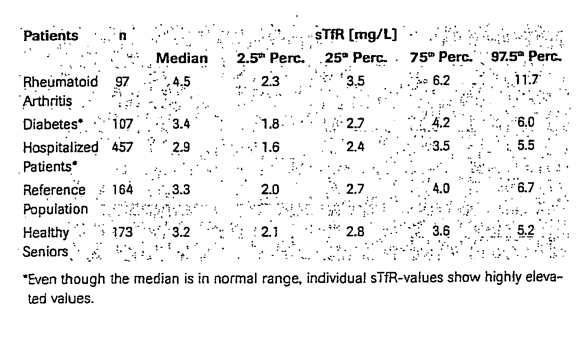

Patients with chronic diseases such as rheumatoid arthritis, renal insufficiency, CAD or diabetes had significantly higher values of sTfR. The determination of sTfR, optionally in combination with ferritin, was found to be a sensitive tool for the assessment of functional iron deficiency in various patient groups. Further, sTfR can be used as an early predictor of risk among patients with coronary syndrome.

### Example 2

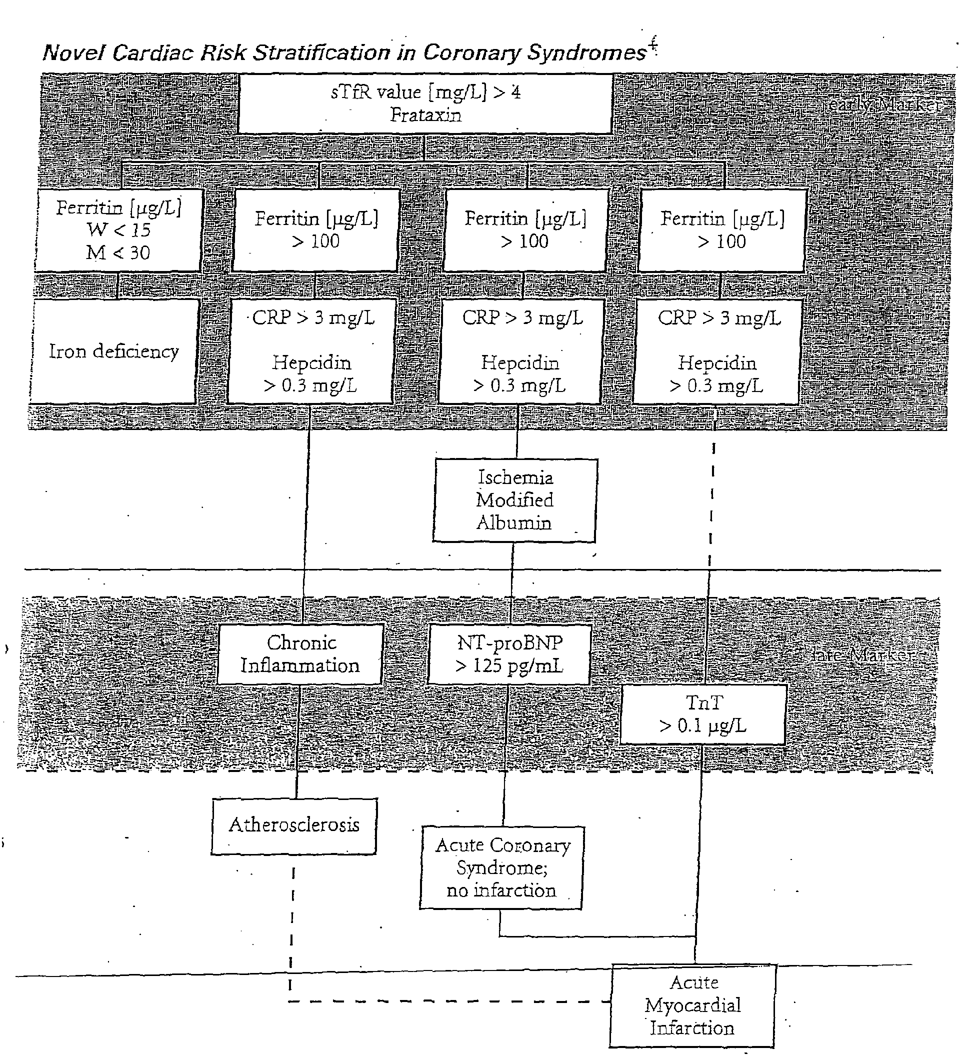

This example shows baseline levels of soluble transferrin receptor for cardiovascular risk prediction obtained from patients with documented coronary artery disease. Further, the results presented allow to place sTfR into perspective of classical risk predictors.

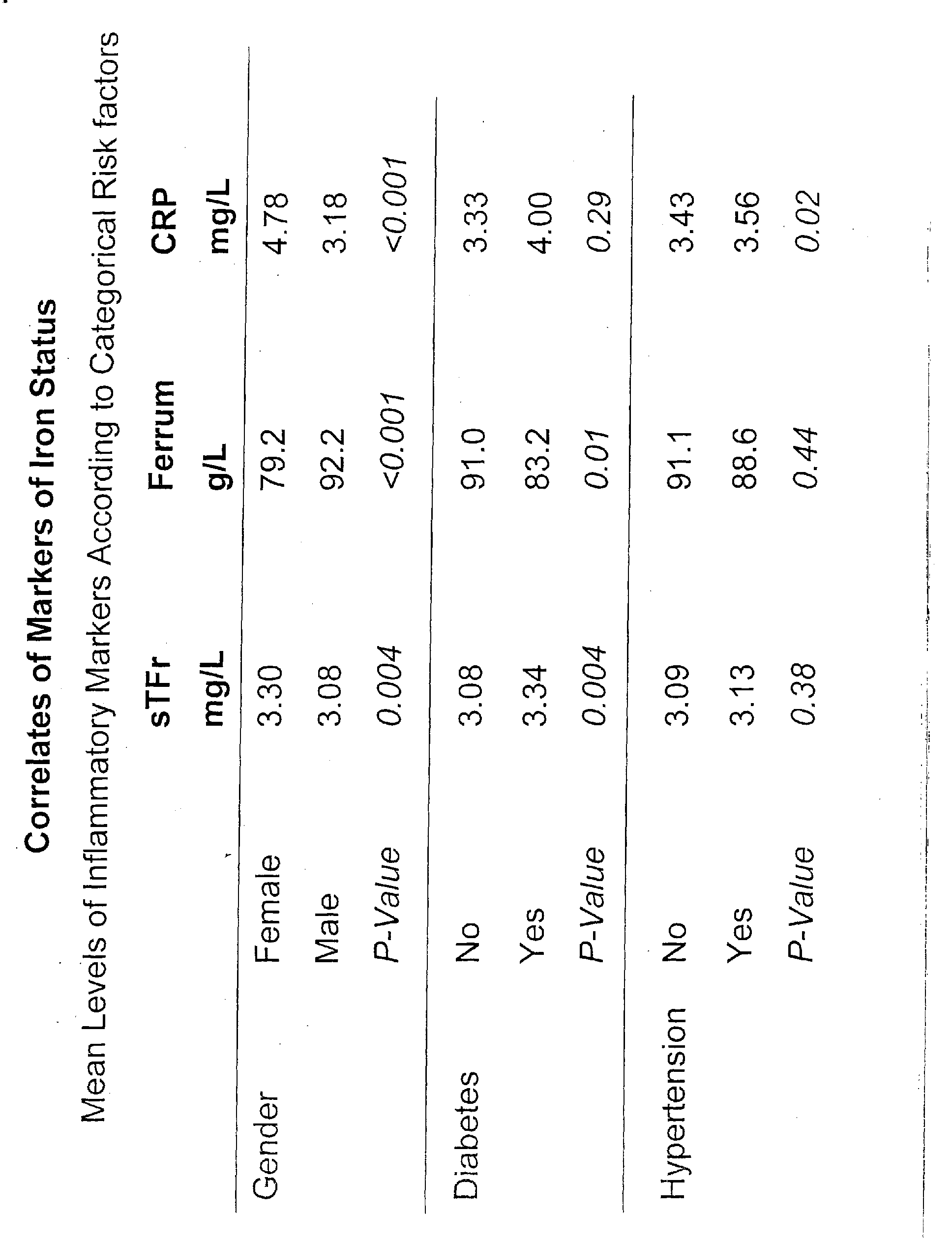

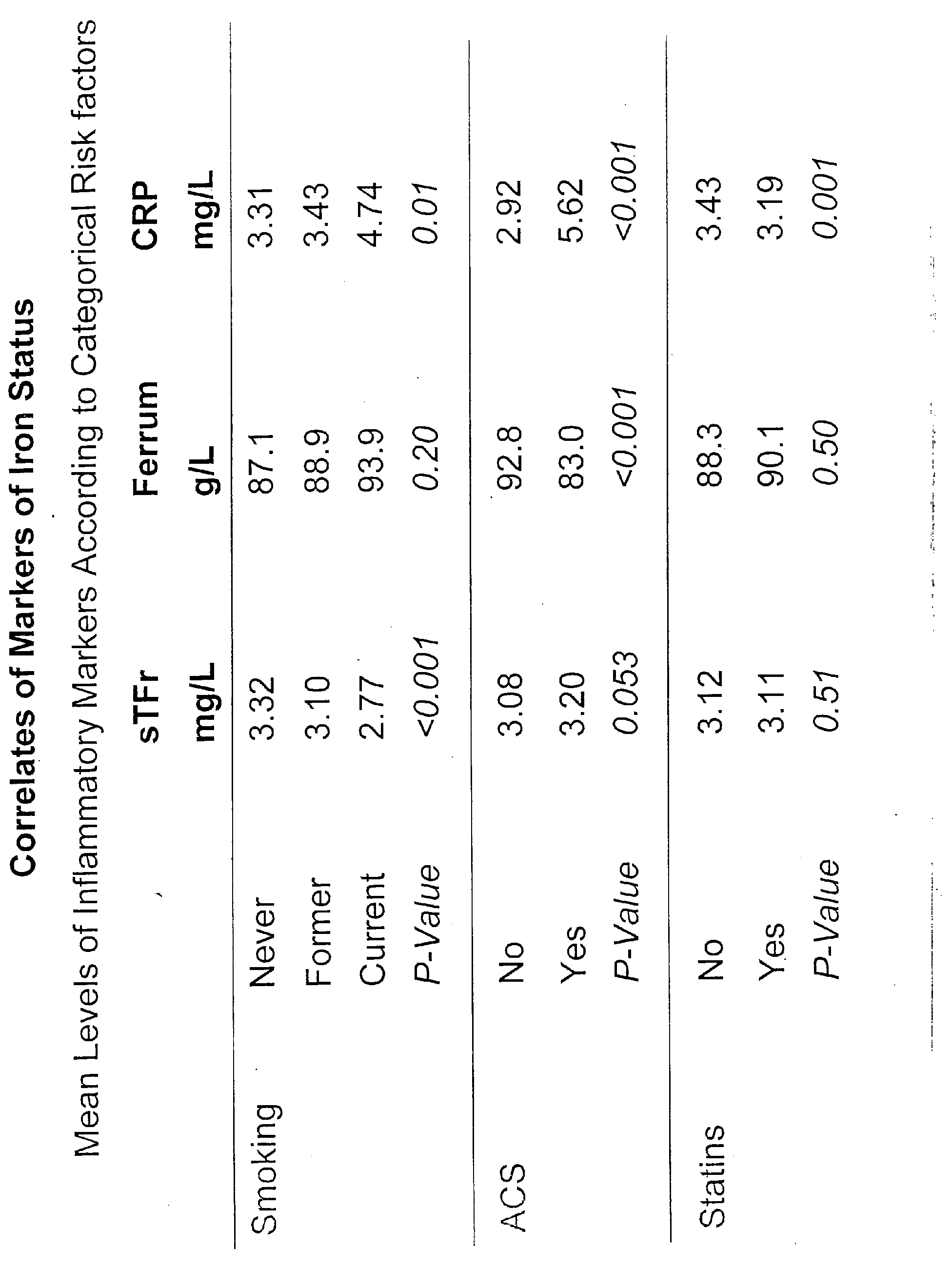

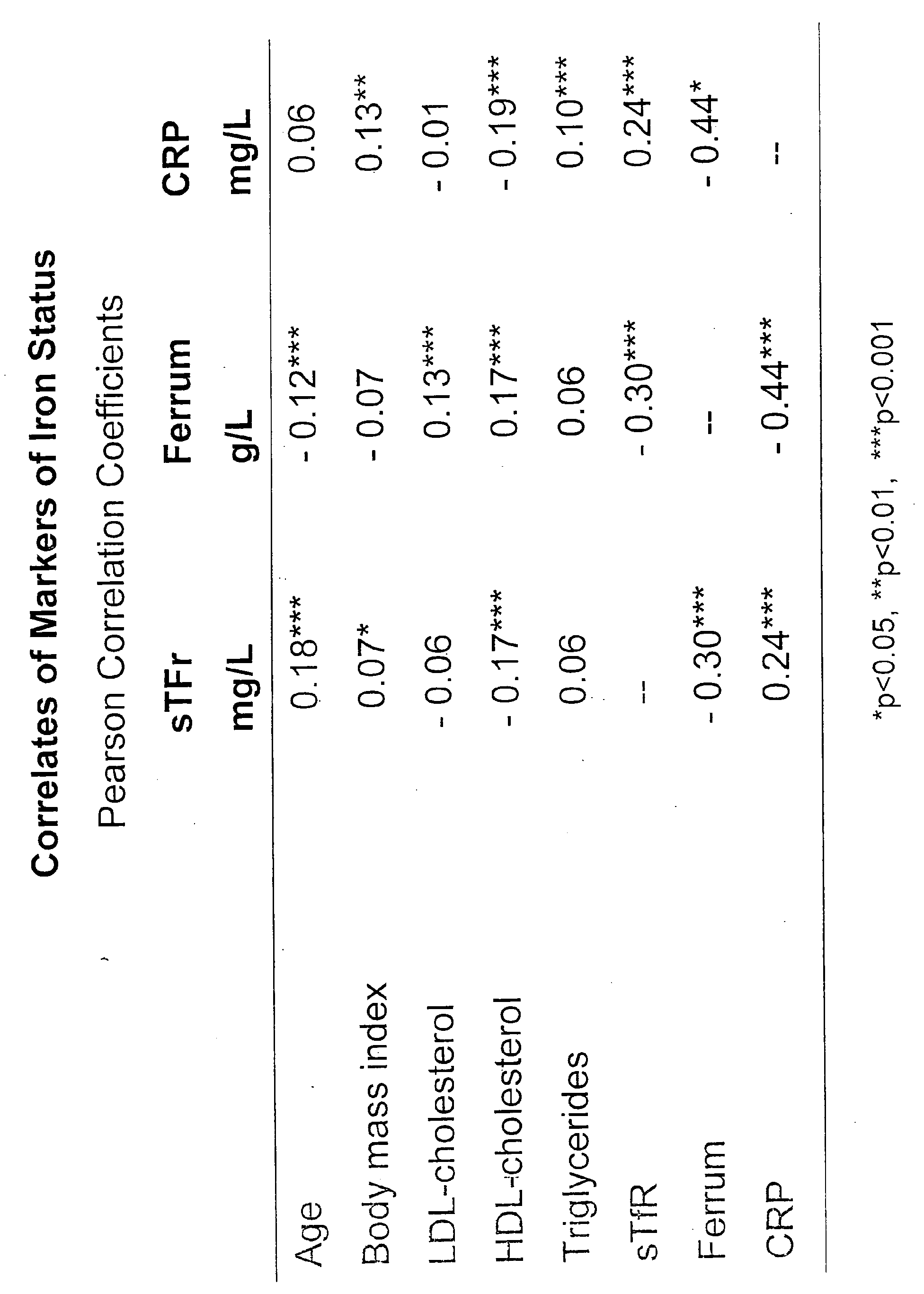

### Example 3

### Clinical assessment of sTfR as an independent risk marker in coronary syndromes

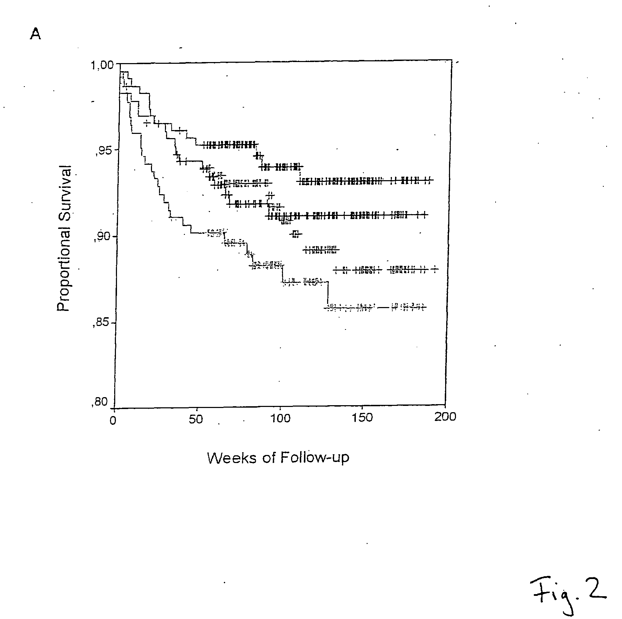

In a further study during 200 weeks with 700 patients, patients with significantly higher values of sTfR > 4mg/L had a lower survival function compared to patients with lower sTfR values. The respective graphs showing proportional survival over weeks of follow-up are shown in Fig.2. The results strongly support the role of soluble transferrin receptor as a novel risk marker for coronary artery disease.

## Claims

1. Use of sTfR (soluble transferrin receptor) or/and frataxin or/and sTfR/log ferritin as a risk marker for coronary syndromes or/and diabetes mellitus.

2. Use according to claim 1, as a risk marker for coronary artery disease (CAD).

3. Use of at least three compounds selected from sTfR, frataxin, CRP, hs-CRP, hepcidin, BNP, preproBNP, NT-proBNP, troponin I or/and troponin T as risk markers for coronary syndromes or/and diabetes mellitus.

4. Use of at least one compound of each of the following groups:
1) sTfR, frataxin, sTfR/log ferritin
2) CRP, in particular hs-CRP, hepcidin,
3) BNP, preproBNP, NT-proBNP, and
4) troponin T, troponin I
or fragments thereof
for providing prognostic information regarding coronary syndromes or/and diabetes mellitus.

5. Use according to any of claims 1 to 4, wherein additionally one or more of the markers of
(i) acute markers and/or specific markers of myocardial injury,
(ii) non-specific markers of myocardial injury related to coagulation
(iii) non-specific markers of myocardial injury related to artherosclerotic plaque rupture, or/and
(iv) non-specific markers of myocardial injury
is/are determined.

6. Use according to claim 5, wherein
group (i) markers are selected from anexin V, also called lipocortin V, endonexin II, calphobindin I, calcium binding protein 33, placental anticoagulant protein I, thromboplastin inhibitor, vascular anticoagulant-α, anchorin CII, B-type natriuretic peptide (BNP), also called brain-type natriuretic peptide, enolase, TnT, Tnl, fTnT, CK, GP, H-FABP, PG AM as well as S-100,
group (ii) markers are selected from plasmin, β-thromboglobulin (β-TG), PF4, FPA, PDGF, prothrombin fragment 1+2, P-selection, thrombin, D-dimer, von Willebrand factor, TF and coagulation cascade,
group (iii) markers are selected from human neutrophil elastase, inducible nitric oxide synthase, lysophosphatidic acid, malondialdehyde-modified low density lipoprotein and members of the matrix metalloproteinase (MMP) family, including MMP-1, MMP-2, MMP-3 and MMP-9, and/or
group (iv) markers are selected from C-reactive protein, interleukin-1β, interleukin-1 receptor antagonist, interleukin-6, monocyte chemotactic protein-1, soluble intercellular adhesion molecule-1, soluble vascular cell adhesion molecule-1, tumor necrosis factor α (TNFα), caspase-3 and hemoglobin α2.

7. Reagent kit for the diagnosis of coronary syndromes or/and diabetes mellitus containing means to determine at least one compound of each of the following groups:
1) sTfR, frataxin, sTfR/log ferritin
2) CRP, in particular, hs-CRP, hepcidin,
3) BNP, preproBNP, NT-proBNP, and
4) troponin T, troponin I.

8. Reagent kit for the diagnosis of coronary syndromes containing means to determine at least sTfR and/or frataxin and/or sTfR/log ferritin and at least one marker of group (i), (ii), III) and/or (iv) as defined in claim 5 or 6.
